# EUROPEAN PATENT SPECIFICATION

(11) **EP 1 751 302 B1**
(45) Date of publication and mention of the grant of the patent: **28.03.2012**
(21) Application number: 05751856.5
(22) Date of filing: 20.05.2005
(51) Int. Cl.: C12Q 1/00, A61B 5/00, G01N 27/30

(54) **ANALYTE SENSORS AND METHODS FOR MAKING AND USING THEM**
ANALYTSENSOREN UND VERFAHREN ZU DEREN HERSTELLUNG UND VERWENDUNG
DETECTEURS D'ANALYTE ET PROCEDES DE FABRICATION ET D'UTILISATION DE CES DETECTEURS

(30) Priority: 04.06.2004 US 861837
(43) Date of publication of application: 14.02.2007
(73) Proprietor: Medtronic MiniMed, Inc., Northridge, CA 91325-1219 (US)
(72) Inventor: SHAH, Rajiv, Palos Verdes, CA 90275 (US); REGHABI, Bahar, Marina del Rey, CA 90292 (US); GOTTLIEB, Rebecca, K., Culver City, CA 90232 (US); HOSS, Udo, Sherman Oaks, CA 91403 (US); MASTROTOTARO, John, Joseph, Los Angeles, CA 90024 (US)
(74) Representative: Ruschke, Hans Edvard
(86) International application number: PCT/US2005/017885
(87) International publication number: WO 2005/121355

(56) References cited:
- US-A- 5 281 324
- US-A- 5 858 186
- US-A1- 2003 100 040
- US-A1- 2004 074 785
- US-B1- 6 497 729
- PATENT ABSTRACTS OF JAPAN vol. 010, no. 019 (P-423), 24 January 1986 (1986-01-24) & JP 60 173452 A (FUJI DENKI SOUGOU KENKYUSHO:KK), 6 September 1985 (1985-09-06)

## Description

### Background of the Invention

### 1. Field of the Invention.

The present invention relates to sensors for the detection and measurement of analytes such as glucose and methods for making and using these sensors.

### 2. Description of Related Art.

The assay of biochemical analytes such as glucose and lactate is important in a variety of clinical contexts. For example, the monitoring of glucose concentrations in fluids of the human body is of particular relevance to diabetes management. Continuously or intermittently operating glucose sensors, including sensors implanted in the human body, are sought for the management of diabetes, for example, for warning of imminent or actual hypoglycemia as well as its avoidance. The monitoring of lactate concentrations in fluids of the human body is useful in, but not limited to, the diagnosis and assessment of a number of medical conditions including trauma, myocardial infarction, congestive heart failure, pulmonary edema and septicemia.

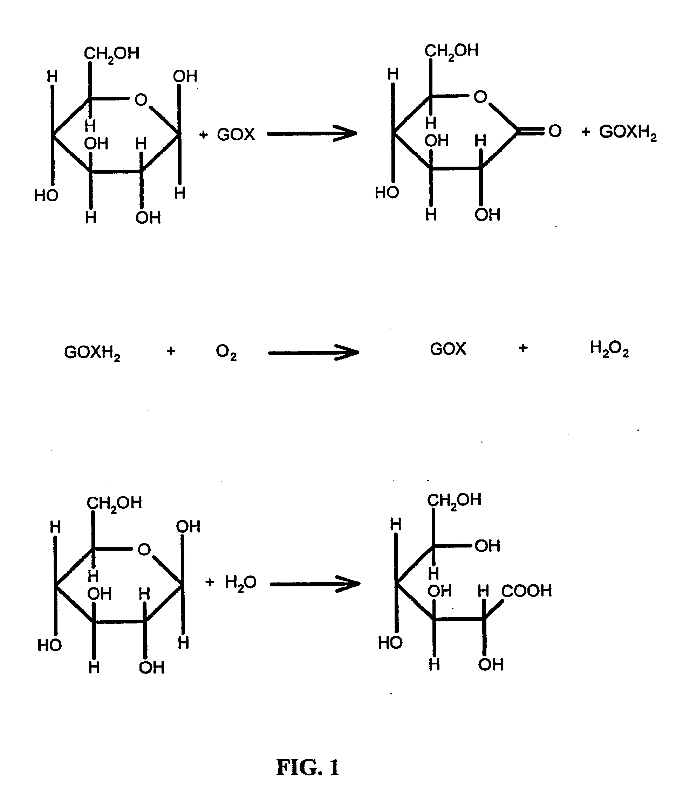

Biomedical measuring devices commonly used by to monitor physiological variables include amperometric sensor devices that utilize electrodes modified with an appropriate enzyme coating. Sensors having such enzyme electrodes enable the user to determine the concentration of various analytes rapidly and with considerable accuracy, for example by utilizing the reaction of an enzyme and an analyte where this reaction utilizes a detectable coreactant and/or produces a detectable reaction product. For example, a number of glucose sensors have been developed that are based on the reaction between glucose and glucose oxidase (GOx) as shown in Figure 1. In this context, the accurate measurement of physiological glucose concentrations using sensors known in the art, typically requires that both oxygen and water be present in excess. As glucose and oxygen diffuse into an immobilized enzyme layer on a sensor, the glucose reacts with oxygen to produce H₂O₂. Glucose can be detected electrochemically using the immobilized enzyme glucose oxidase coupled to oxygen and/or hydrogen peroxide-sensitive electrodes. The reaction results in a reduction in oxygen and the production of hydrogen peroxide proportional to the concentration of glucose in the sample medium. A typical device is composed of (but not limited to) at least two detecting electrodes, or at least one detecting electrode and a reference signal source, to sense the concentration of oxygen or hydrogen peroxide in the presence and absence of enzyme reaction. Additionally, the complete monitoring system typically contains an electronic sensing and control means for determining the difference in the concentration of the substances of interest. From this difference, the concentration of analytes such as glucose can be determined.

A wide variety of such analyte sensors as well as methods for making and using such sensors are known in the art. Examples of such sensors, sensor sets and methods for their production are described, for example, in U.S. Patent Nos. 5,390,691, 5,391, 250, 5,482,473, 5,299,571, 5,568,806 as well as PCT International Publication Numbers WO 01/58348, WO 03/034902, WO 03/035117, WO 03/035891, WO 03/023388, WO 03/022128, WO 03/022352, WO 03/023708, WO 03/036255, WO03/036310 and WO 03/074107

U.S. Patent No. 6,497,729 describes implants to human and animal bodies, especially apparatus and methods for controlling tissue/implant interactions. A tissue/implant interface comprises an implant and a bioactive polymer layer adjacent at least a portion of the outer surface of the implant; the polymer layer contains at least one tissue response modifier covalently attached to the polymer layer or entrapped within the polymer layer in a quantity effective to control the tissue response at the side of implantation. Preferably, the at least one tissue response modifier controls inflammation, fibrosis, and/or neovascularization.

Another sensor is disclosed in U.S. Patent No. 5,281,324. This measuring sensor detects gaseous components which operate in the diffusion limit current region. The measuring sensor includes a support body and has an electrolyte chamber partitioned by a diffusion membrane form the ambient containing the gaseous components. While a number of sensor designs and processes for making such sensors are known in the art, there continues to be a need for sensors having improved characteristics such as enhanced stability, longevity, linearity and regularity, as well as optimized signal to noise ratios. There is also a need for the identification of the methods and processes that allow for the generation of sensors having these optimized qualities. The present invention fulfills these needs and provides further related advantages.

### Summary of the Invention

Embodiments of the invention disclosed herein provide analyte sensors of the type used, for example, in subcutaneous or transcutaneous monitoring of blood glucose levels in a diabetic patient as defined in claim 1 Embodiments of the invention disclosed herein further provide analyte sensors of the type used, for example, in a variety of clinical contexts such as with dialysis and/or extra corporeal membrane oxygenation protocols. More specifically, the disclosure provided herein teaches optimized analyte sensor designs and method for making such sensors as defined in claim19. Preferred analyte sensors of the invention include very thin analyte sensing layers that typically comprise enzymes such as glucose oxidase, lactate oxidase and the like. In addition, the analyte sensors of the invention preferably include one or more layers comprising a silane which serve to promote the adhesion between the layers of the analyte sensor. Surprisingly, analyte sensors that incorporate silane adhesion promoting layers and other elements disclosed herein have a number of superior qualities including enhanced stability, longevity, linearity and regularity, as well as improved signal to noise ratios.

The invention disclosed herein has a number of embodiments. A typical embodiment of the invention is an analyte sensor apparatus designed for implantation within a mammal. The analyte sensor apparatus includes as defined in claim 1 a base layer and a conductive layer disposed upon the base layer wherein the conductive layer includes a working electrode and preferably a reference electrode and a counter electrode. In this embodiment of the invention, an analyte sensing layer is disposed on the conductive layer. Typically, the analyte sensing layer comprises a composition that detectably alters the electrical current at the working electrode in the conductive layer in the presence of an analyte. Illustrative example of such compositions include enzymes such as glucose oxidase, glucose dehydrogenase, lactate oxidase, hexokinase and lactose dehydrogenase or the like (e.g. any other protein and/or polymer and/or a combination thereof that stabilizes the enzyme layer). This embodiment of the invention includes a protein layer disposed on the analyte sensing layer, with this protein layer typically including a carrier protein such as bovine serum albumin or human serum albumin or the like. In this embodiment, an adhesion promoting layer is disposed on the protein layer. Preferably this adhesion promoting layer includes a silane composition selected for its ability to enhance the stability of the sensor structure, for example γ-aminopropyltrimethoxysilane.

This embodiment also includes an analyte modulating layer disposed above the analyte sensing layer, wherein the analyte modulating layer modulates the diffusion of the analyte therethrough, for example a glucose limiting membrane. Preferably this embodiment also includes a insulative cover layer disposed on at least a portion of the analyte modulating layer, wherein the cover layer further includes an aperture that exposes at least a portion of the analyte modulating layer to a solution comprising the analyte to be sensed. Preferably the analyte sensor apparatus is designed to function via anodic polarization such that the alteration in current can be detected at the working electrode (anode) in the conductive layer of the analyte sensor apparatus; and the alteration in current that can be detected at this working anode can be correlated with the concentration of the analyte.

As described in detail below, the various layers of the sensor can exhibit a variety of different characteristics which can be manipulated according to the preferred design of the sensor. For example, the analyte sensing layer can comprise an enzyme selected from the group consisting of glucose oxidase, glucose dehydrogenase, lactate oxidase, hexokinase and lactose dehydrogenase. Alternatively, the analyte sensing layer can comprise an antibody or other analyte sensing molecule. Preferably analyte sensing layer is a thickness selected from the group consisting of less than 1, 0.5, 0.25 and 0.1 microns and comprises a carrier protein in a substantially fixed ratio with an enzyme, wherein the enzyme and the carrier protein are distributed in a substantially uniform manner throughout the enzyme layer.

In one illustrative embodiment of the invention, the enzyme in the analyte sensing layer is glucose oxidase and the analyte sensor apparatus is capable of sensing glucose levels in the mammal. In such sensor embodiments, the current at the working electrode in the conductive layer is altered by hydrogen peroxide that is generated from the enzymatic reaction between glucose and oxygen via glucose oxidase. In an alternative illustrative embodiment of the invention, the enzyme in the analyte sensing layer is lactate oxidase and the analyte sensor apparatus is capable of sensing lactate levels in the mammal. In such sensor embodiments, the current at the working electrode in the conductive layer is altered by hydrogen peroxide that is generated from the enzymatic reaction between lactate and oxygen via lactate oxidase.

Certain analyte sensors having the structure discussed above have a number of highly desirable characteristics. For example, certain analyte sensor apparatus embodiments are suitable for implantation in the mammal for a time period of greater than 30 days and up to 12 months or more. Moreover, certain analyte sensor apparatus embodiments can sense an alteration in current in response to exposure to the analyte present in the body of the mammal that can be detected via a device such as an amperemeter within 15, 10, 5 or 2 minutes of the analyte contacting the sensor. In addition, certain analyte sensor apparatus embodiments disclosed herein are suitable for implantation in the mammal in a non-vascular space. Finally, as discussed in detail below, the characteristics of the elements used in certain embodiments of the invention disclosed herein allow for a wider range of geometrical configurations (e.g. small planar sensor configurations) than existing sensors in the art.

Certain analyte sensors having the structure discussed above have a number of highly desirable characteristics which allow for a variety of methods for sensing analytes in a mammal. For example in such methods, the analyte sensor apparatus implanted in the mammal functions to sense an analyte within the body of a mammal for more than 1, 2, 3, 4, 5, or 6 months. Preferably, the analyte sensor apparatus so implanted in the mammal senses an alteration in current in response to an analyte within 15, 10, 5 or 2 minutes of the analyte contracting the sensor. In such methods, the sensors can be implanted into a variety of locations within the body of the mammal, for example in both vascular and non-vascular spaces.

The invention also provides additional articles of manufacture including sensor sets and kits. In one such embodiment of the invention, a kit useful for the sensing an analyte as is described above, is provided. The kit typically comprises a container, a label and a sensor as described above. The typical embodiment is a kit comprising a container and, within the container, an analyte sensor apparatus having a design as disclosed herein and instructions for using the analyte sensor apparatus as defined in claim 29.

### Brief Description of the Figures

Figure 1 provides a schematic of the well known reaction between glucose and glucose oxidase. As shown in a stepwise manner, this reaction involves glucose oxidase (GOx), glucose and oxygen in water. In the reductive half of the reaction, two protons and electrons are transferred from β-D-glucose to the enzyme yielding d-gluconolactone In the oxidative half of the reaction, the enzyme is oxidized by molecular oxygen yielding hydrogen peroxide. The d-gluconolactone then reacts with water to hydrolyze the lactone ring and produce gluconic acid. In certain electrochemical sensors of the invention, the hydrogen peroxide produced by this reaction is oxidized at the working electrode (H₂O₂ → 2H+ + O₂ + 2e⁻).
Figure 2 provides a diagrammatic view of a typical analyte sensor configuration of the current invention.
Figure 3 provides an overview (upper) and cross sectional views (lower) of a relatively flat "ribbon" type sensor configuration that can be made with the analyte sensor apparatus.
Figures 4A and 4B illustrate various sensor configurations that include multiple conductive elements such as multiple working, counter and reference electrodes. Figure 4B illustrates a sensor design with 7 vias and 4 working electrodes where W = working electrode (+), C = counter electrode (-) and R = reference electrode.

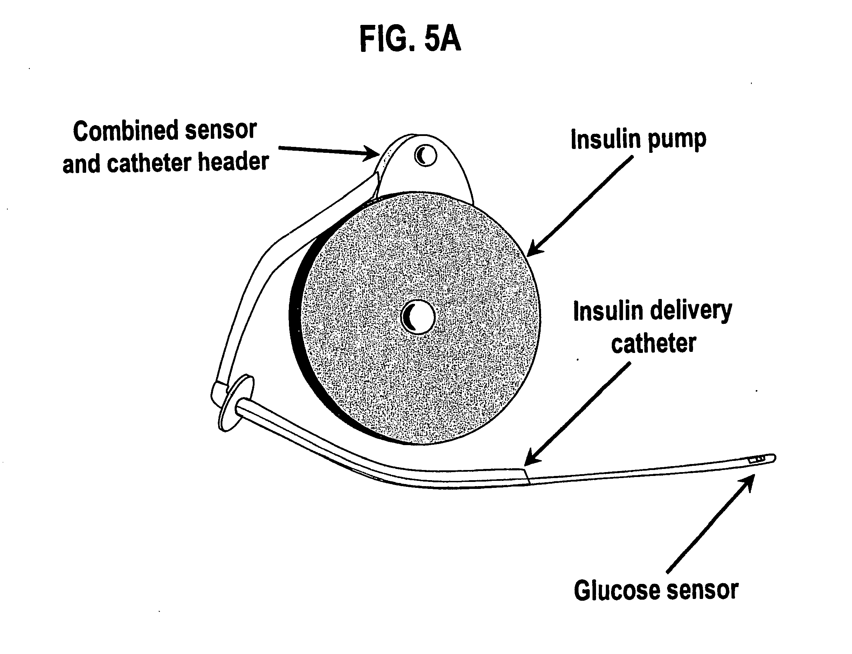

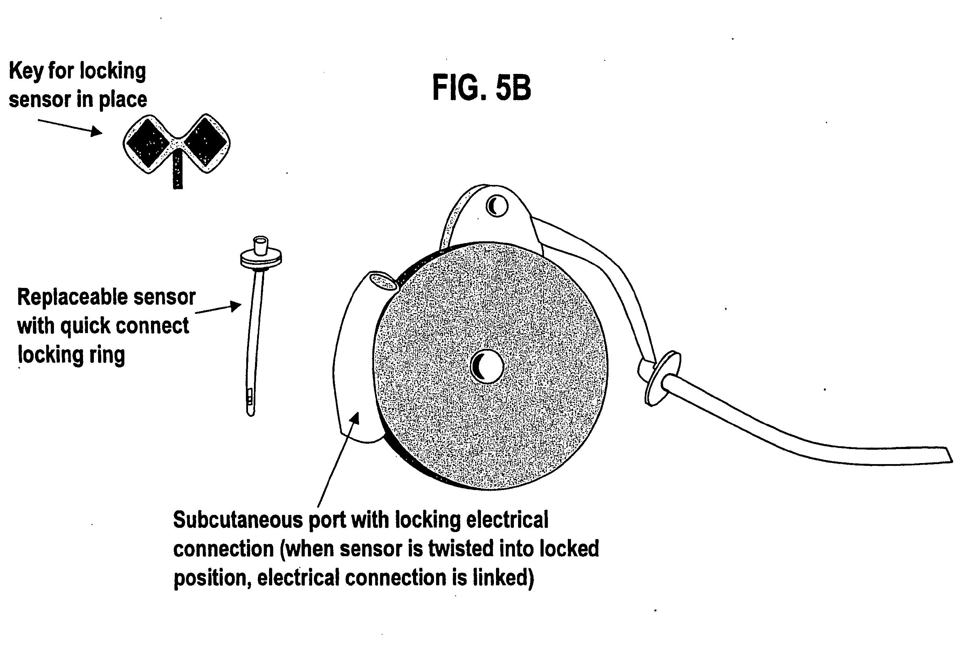

Figure 5A provides an illustration of how the analyte sensors of the invention can be coupled with other medical devices such as insulin delivery catheters, combined sensor and catheter header and medication infusion pumps. Figure 5B provides an illustration of a variation of this scheme where replaceable analyte sensors of the invention can be coupled with other medical devices such as medication infusion pumps, for example by the use of a port coupled to the medical device (e.g. a subcutaneous port with a locking electrical connection). The design provided in Figure 5B, illustrates a replaceable sensor integrated with a port on the pump, wherein the port is a subcutaneous port with a locking electrical connection (when sensor is twisted into locked position, electrical connection is linked). Also shown in Figure 5B is a replaceable sensor with quick connect locking ring and a key for locking the sensor in place.

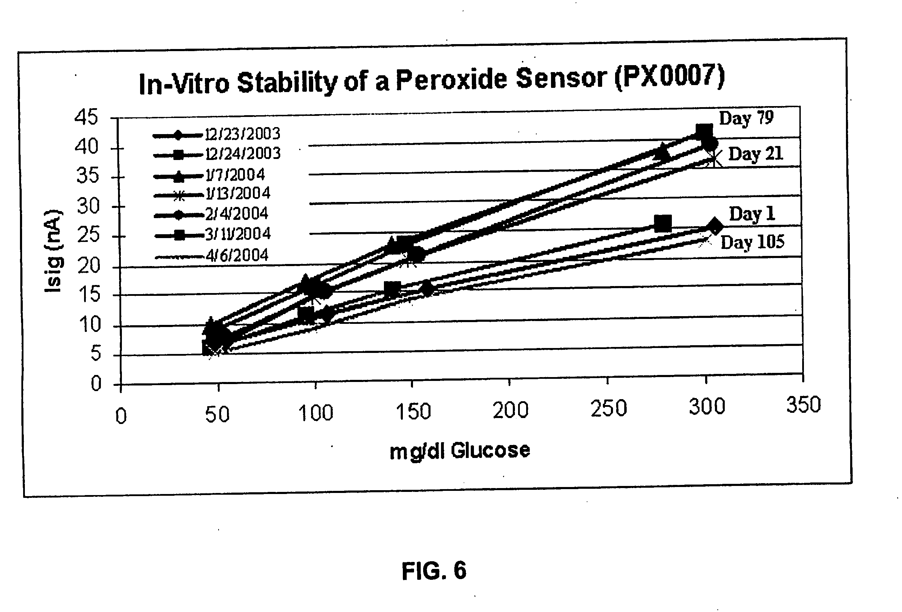

Figure 6 provides a graphic representation of properties of a peroxide based glucose sensor embodiment of the present invention which utilizes glucose oxidase in the analyte sensing layer and illustrates the long term stability of the peroxide sensor.

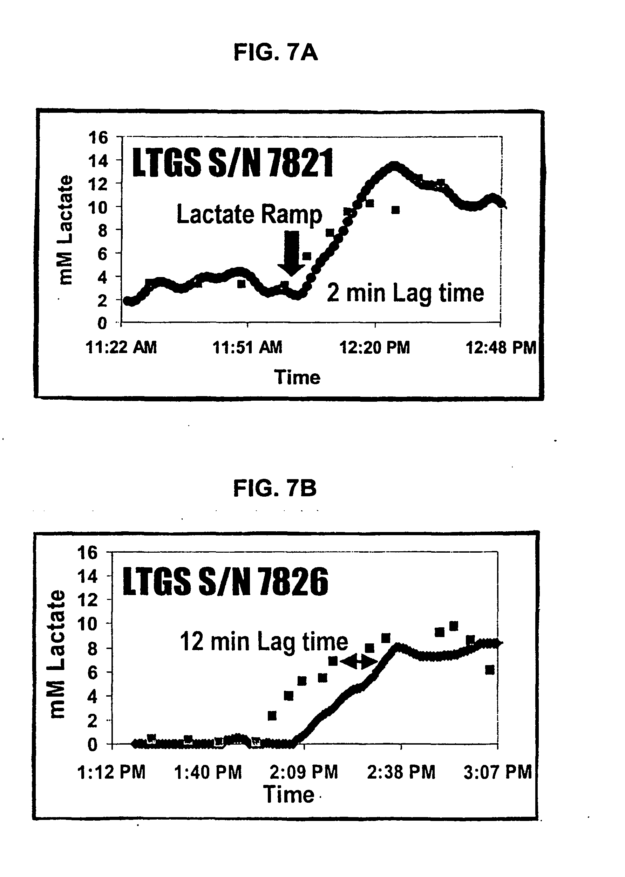

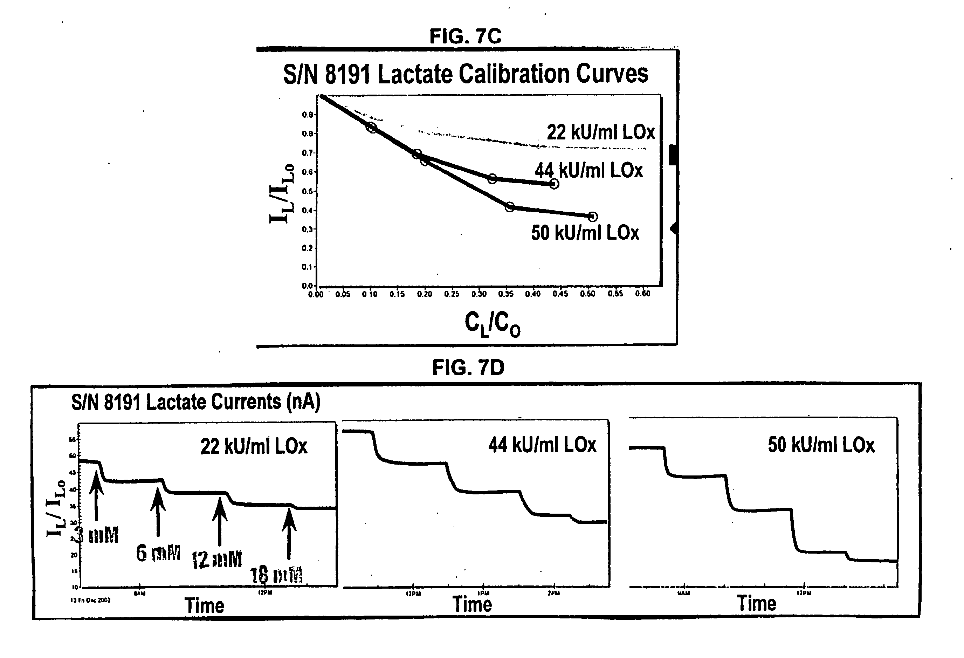

Figures 7A-7D provides graphic representations of properties of a long term oxygen based lactate sensor embodiment of the present invention (in a catheter like device configuration) which utilizes lactate oxidase in the analyte sensing layer. Figures 7A and 7B data is derived from in-vivo canine studies. Figures 7C and 7D show that lactate oxidase (LOx) formulations exhibit a highly desirable characteristics including a dynamic range and sensitivity.

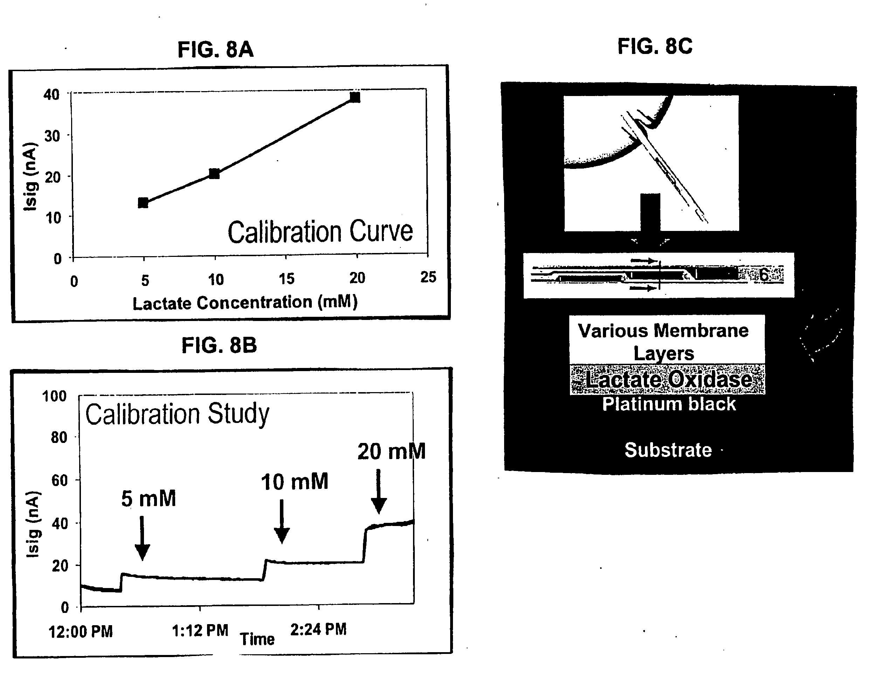

Figure 8a-8C provides an image of the in-vitro calibration of a peroxide based sensor of lactate. Figure 8A provides a calibration study. Figure 8B provides a calibration curve. Figure 8C provides a schematic of the sensor.

### Detailed Description of the Preferred Embodiments

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art. As appropriate, procedures involving the use of commercially available kits and reagents are generally carried out in accordance with manufacturer defined protocols and/or parameters unless otherwise noted.

Embodiments of the invention disclosed herein provide sensors of the type used, for example, in subcutaneous or transcutaneous monitoring of blood glucose levels in a diabetic patient. The disclosure further provides methods for making and using such sensors. While preferred embodiments of the invention pertain to glucose and/or lactate sensors, a variety of the elements disclosed herein (e.g. thin enzyme coatings) can be adapted for use with any one of the wide variety of sensors known in the art. The analyte sensor elements, architectures and methods for making and using these elements that are disclosed herein can be used to establish a variety of layered sensor structures. Such sensors of the invention exhibit a surprising degree of flexibility and versatility, characteristic which allow a wide variety of sensor configurations to be designed to examine a wide variety of analyte species. In typical embodiments of the present invention, the transduction of the analyte concentration into a processable signal is by electrochemical means. These transducers may include any of a wide variety of amperometric, potentiometric, or conductimetric base sensors known in the art. Moreover, the microfabrication sensor techniques and materials of the instant invention may be applied to other types of transducers (e.g., acoustic wave sensing devices, thermistors, gas-sensing electrodes, field-effect transistors, optical and evanescent field wave guides, and the like) fabricated in a substantially nonplanar, or alternatively, a substantially planar manner. A useful discussion and tabulation of transducers which may be exploited in a biosensor as well as the kinds of analytical applications in which each type of transducer or biosensor, in general, may be utilized is found in an article by Christopher R. Lowe in Trends in Biotech. 1984, 2(3), 59-65.

Specific aspects of the invention are discussed in detail in the following sections.

### I. TYPICAL ANALYTE SENSORS, SENSOR ELEMENTS AND SENSOR CONFIGURATIONS OF THE INVENTION

### A. DIAGRAMMATIC ILLUSTRATION OF TYPICAL SENSOR CONFIGURATION

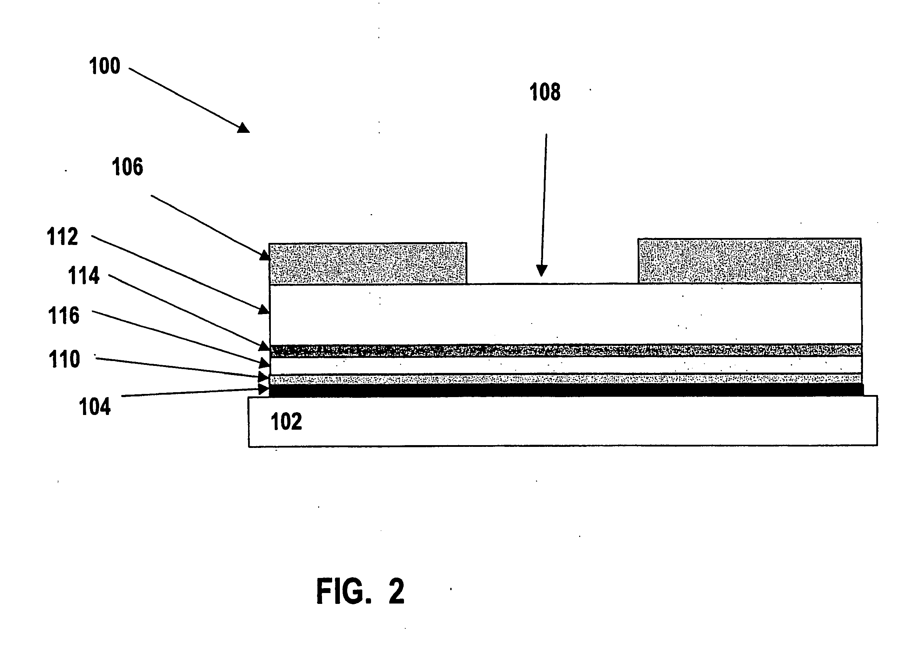

FIG. 2 illustrates a cross-section of a typical sensor structure 100 of the present invention. The sensor is formed from a plurality of layers of various conductive and non-conductive constituents disposed on each other according to a method of the invention to produce a sensor structure. The embodiment shown in FIG. 2 includes a base layer 102 to support the sensor 100. The base layer 102 can be made of a material such as a ceramic or polyimide substrate, which may be self-supporting or further supported by another material as is known in the art. Embodiments of the invention include a conductive layer 104 which is disposed on the base layer 102. Typically the conductive layer 104 comprises one or more electrodes. An operating sensor 100 typically includes a plurality of electrodes such as a working electrode, a counter electrode and a reference electrode. Other embodiments may also include an electrode that performs multiple functions, for example one that functions as both as a reference and a counter electrode. Still other embodiments may utilize a separate reference element not formed on the sensor. Typically these electrodes are electrically isolated from each other, while situated in close proximity to one another.

As discussed in detail below, the conductive layer 104 can be applied using many known techniques and materials. The electrical circuit of the sensor is typically defined by etching the disposed conductive layer 104 into a desired pattern of conductive paths. A typical electrical circuit for the sensor 100 comprises two or more adjacent conductive paths with regions at a proximal end to form contact pads and regions at a distal end to form sensor electrodes. An electrically insulating cover layer 106 such as a polymer coating is typically disposed on portions of the sensor 100. Acceptable polymer coatings for use as the insulating protective cover layer 106 can include, but are not limited to, non-toxic biocompatible polymers such as silicone compounds, polyimides, biocompatible solder masks, epoxy acrylate copolymers, or the like. In the sensors of the present invention, one or more exposed regions or apertures 108 can be made through the cover layer 106 to open the conductive layer 104 to the external environment and to for example allow an analyte such as glucose to permeate the layers of the sensor and be sensed by the sensing elements. Apertures 108 can be formed by a number of techniques, including laser ablation, chemical milling or etching or photolithographic development or the like. In certain embodiments of the invention, during manufacture, a secondary photoresist can also be applied to the protective layer 106 to define the regions of the protective layer to be removed to form the aperture(s) 108. The exposed electrodes and/or contact pads can also undergo secondary processing (e.g. through the apertures 108), such as additional plating processing, to prepare the surfaces and/or strengthen the conductive regions.

In the sensor configuration shown in FIG. 2, an analyte sensing layer 110 (which is preferably a sensor chemistry layer, meaning that materials in this layer undergo a chemical reaction to produce a signal that can be sensed by the conductive layer) is disposed on one or more of the exposed electrodes of the conductive layer 104. Preferably, the sensor chemistry layer 110 is an enzyme layer. Most preferably, the sensor chemistry layer 110 comprises an enzyme capable of producing utilizing oxygen and/or hydrogen peroxide, for example the enzyme glucose oxidase. Optionally the enzyme in the sensor chemistry layer is combined with a second carrier protein such as human serum albumin, bovine serum albumin or the like. In an illustrative embodiment, an enzyme such as glucose oxidase in the sensor chemistry layer 110 reacts with glucose to produce hydrogen peroxide, a compound which then modulates a current at an electrode. As this modulation of current depends on the concentration of hydrogen peroxide, and the concentration of hydrogen peroxide correlates to the concentration of glucose, the concentration of glucose can be determined by monitoring this modulation in the current. In a specific embodiment of the invention, the hydrogen peroxide is oxidized at a working electrode which is an anode (also termed herein the anodic working electrode), with the resulting current being proportional to the hydrogen peroxide concentration. Such modulations in the current caused by changing hydrogen peroxide concentrations can by monitored by any one of a variety of sensor detector apparatuses such as a universal sensor amperometric biosensor detector or one of the other variety of similar devices known in the art such as glucose monitoring devices produced by Medtronic MiniMed.

The analyte sensing layer 110 can be applied over portions of the conductive layer or over the entire region of the conductive layer. Typically the analyte sensing layer 110 is disposed on the working electrode which can be the anode or the cathode. Optionally, the analyte sensing layer 110 is also disposed on a counter and/or reference electrode. Typically, analyte sensing layer 110 is relatively thin as compared to those found in sensors previously described in the art, and is for example, preferably less than 1, 0.5, 0.25 or 0.1 microns in thickness. As discussed in detail below, preferred methods for generating a thin analyte sensing layer 110 include spin coating processes, dip and dry processes, low shear spraying processes, ink-jet printing processes, silk screen processes and the like. Most preferably the thin analyte sensing layer 110 is applied using a spin coating process.

Typically, the analyte sensing layer 110 is coated with one or more additional layers. Optionally, the one or more additional layers includes a protein layer 116 disposed upon the analyte sensing layer 110. Typically, the protein layer 116 comprises a protein such as albumin or the like. Preferably, the protein layer 116 comprises human serum albumin. In preferred embodiments of the invention, an additional layer includes an analyte modulating layer 112 that is disposed above the analyte sensing layer 110 to regulate analyte contact with the analyte sensing layer 110. For example, the analyte modulating membrane layer 112 can comprise a glucose limiting membrane, which regulates the amount of glucose that contacts an enzyme such as glucose oxidase that is present in the analyte sensing layer. Such glucose limiting membranes can be made from a wide variety of materials known to be suitable for such purposes, e.g., silicone compounds such as polydimethyl siloxanes, polyurethanes, polyurea cellulose acetates, Nafion, polyester sulfonic acids (e.g. Kodak AQ), hydrogels or any other suitable hydrophilic membranes known to those skilled in the art.

In typical embodiments of the invention, an adhesion promoter layer 114 is disposed between the analyte modulating layer 112 and the analyte sensing layer 110 as shown in FIG. 2 in order to facilitate their contact and/or adhesion. In a specific embodiment of the invention, an adhesion promoter layer 114 is disposed between the analyte modulating layer 112 and the protein layer 116 as shown in FIG. 2 in order to facilitate their contact and/or adhesion. The adhesion promoter layer 114 can be made from any one of a wide variety of materials known in the art to facilitate the bonding between such layers. Preferably, the adhesion promoter layer 114 comprises a silane compound. In alternative embodiments, protein or like molecules in the analyte sensing layer 110 can be sufficiently crosslinked or otherwise prepared to allow the analyte modulating membrane layer 112 to be disposed in direct contact with the analyte sensing layer 110 in the absence of an adhesion promoter layer 114.

### B. TYPICAL ANALYTE SENSOR LAYERS

### BASE LAYER

Sensors of the invention typically include a base layer (see, e.g. element 102 in Figure 2). The term "base layer" is used herein according to art accepted terminology and refers to the layer in the apparatus that typically provides a supporting matrix for the plurality of layers that are stacked on top of one another and comprise the functioning sensor. In a preferred form, the base layer comprises a thin film sheet of insulative (e.g. electrically insulative and/or water impermeable) material. This base layer can be made of a wide variety of materials having desirable qualities such as water impermeability and hermeticity. Preferred materials include ceramic and polyimide substrates or the like. The base layer may be self-supporting or further supported by another material as is known in the art. In one embodiment of the sensor configuration shown in Figure 2, the base layer 102 comprises a ceramic. In an illustrative embodiment, the ceramic base comprises a composition that is predominantly Al₂O₃ (e.g. 96%). The use of alumina as an insulating base layer for use with implantable devices is disclosed in U.S. Pat. Nos. 4,940,858, 4,678,868 and 6,472,122 which are incorporated herein by reference. The base layers of the invention can further include other elements known in the art, for example hermetical vias (see, e.g. WO 03/023388). Depending upon the specific sensor design, the base layer can be relatively thick layer (e.g. thicker than 25 microns). Alternatively, one can utilize a nonconductive ceramic, such as alumina, in thin layers, e.g., less than about 25 microns.

### CONDUCTIVE LAYER

The electrochemical sensors of the invention typically include a conductive layer disposed upon the base layer that includes at least one electrode for contacting an analyte or its byproduct (e.g. oxygen and/or hydrogen peroxide) to be assayed (see, e.g. element 104 in Figure 2). The term "conductive layer" is used herein according to art accepted terminology and refers to electrically conductive sensor elements such as electrodes which are capable of measuring and a detectable signal and conducting this to a detection apparatus. An illustrative example of this is a conductive layer that can measure an increase or decrease in current in response to exposure to a stimuli such as the change in the concentration of an analyte or its byproduct as compared to a reference electrode that does not experience the change in the concentration of the analyte, a coreactant (e.g. oxygen) used when the analyte interacts with a composition (e.g. the enzyme glucose oxidase) present in analyte sensing layer 110 or the reaction product of this interaction (e.g. hydrogen peroxide). Illustrative examples of such elements include electrodes which are capable of producing a variable detectable signals in the presence of variable concentrations of molecules such as hydrogen peroxide or oxygen. Typically one of these electrodes in the conductive layer is a working electrode, which can be made from non-corroding metal or carbon. A carbon working electrode may be vitreous or graphitic and can be made from a solid or a paste. A metallic working electrode may be made from platinum group metals, including palladium or gold, or a non-corroding metallically conducting oxide, such as ruthenium dioxide. Alternatively the electrode may comprise a silver/silver chloride electrode composition. The working electrode may be a wire or a thin conducting film applied to a substrate, for example, by coating or printing. Typically, only a portion of the surface of the metallic or carbon conductor is in electrolytic contact with the analyte-containing solution. This portion is called the working surface of the electrode. The remaining surface of the electrode is typically isolated from the solution by an electrically insulating cover layer 106. Examples of useful materials for generating this protective cover layer 106 include polymers such as polyimides, polytetrafluoroethylene, polyhexafluoropropylene and silicones such as polysiloxanes.

In addition to the working electrode, the analyte sensors of the invention typically include a reference electrode or a combined reference and counter electrode (also termed a quasi-reference electrode or a counter/reference electrode). If the sensor does not have a counter/reference electrode then it may include a separate counter electrode, which may be made from the same or different materials as the working electrode. Typical sensors of the present invention have one or more working electrodes and one or more counter, reference, and/or counter/reference electrodes. One embodiment of the sensor of the present invention has two, three or four or more working electrodes. These working electrodes in he sensor may be integrally connected or they may be kept separate.

Typically, for in vivo use the analyte sensors of the present invention are implanted subcutaneously in the skin of a mammal for direct contact with the body fluids of the mammal, such as blood. Alternatively the sensors can be implanted into other regions within the body of a mammal such as in the Intraperotineal space. When multiple working electrodes are used, they may be implanted together or at different positions in the body. The counter, reference, and/or counter/reference electrodes may also be implanted either proximate to the working electrode(s) or at other positions within the body of the mammal.

### ANALYTE SENSING LAYER

The electrochemical sensors of the invention include a analyte sensing layer disposed on the electrodes of the sensor (see, e.g. element 110 in Figure 2). The term "analyte sensing layer" is used herein according to art accepted terminology and refers to a layer comprising a material that is capable of recognizing or reacting with an analyte whose presence is to be detected by the analyte sensor apparatus. Typically this material in the analyte sensing layer produces a detectable signal after interacting with the analyte to be sensed, typically via the electrodes of the conductive layer. In this regard the analyte sensing layer and the electrodes of the conductive layer work in combination to produce the electrical signal that is read by an apparatus associated with the analyte sensor. Typically, the analyte sensing layer comprises an enzyme capable of reacting with and/or producing a molecule whose change in concentration can be measured by measuring the change in the current at an electrode of the conductive layer (e.g. oxygen and/or hydrogen peroxide), for example the enzyme glucose oxidase. An enzyme capable of producing a molecule such as hydrogen peroxide can be disposed on the electrodes according to a number of processes known in the art. The analyte sensing layer can coat all or a portion of the various electrodes of the sensor. In this context, the analyte sensing layer may coat the electrodes to an equivalent degree. Alternatively the analyte sensing layer may coat different electrodes to different degrees, with for example the coated surface of the working electrode being larger than the coated surface of the counter and/or reference electrode.

Typical sensor embodiments of this element of the invention utilize an enzyme (e.g. glucose oxidase) that has been combined with a second protein (e.g. albumin) in a fixed ratio (e.g. one that is typically optimized for glucose oxidase stabilizing properties) and then applied on the surface of an electrode to form a thin enzyme layer. In a typical embodiment, the analyte sensing layer comprises a GOx and HSA mixture. A typical embodiments of a analyte sensing layer having GOx, the GOx reacts with glucose present in the sensing environment (e.g. the body of a mammal) and generates hydrogen peroxide according the reaction shown in Figure 1, wherein the hydrogen peroxide so generated is anodically detected at the working electrode in the conductive layer. As discussed for example in U.S. Patent Application Serial Number 10/273,767 extremely thin sensor chemistry layers are preferred and can be applied to the surface of the electrode matrix by processes known in the art such as spin coating. In an illustrative embodiment, a glucose oxidase/albumin is prepared in a physiological solution (e.g., phosphate buffered saline at neutral pH) with the albumin being present in an range of about .5%-10% by weight. Optionally the stabilized glucose oxidase layer that is formed on the analyte sensing layer is very thin as compared to those previously described in the art, for example less than 2, 1, 0.5, 0.25 or 0.1 microns in thickness. One illustrative embodiment of the invention utilizes a stabilized glucose oxidase layer for coating the surface of an electrode wherein the glucose oxidase is mixed with a carrier protein in a fixed ratio within the layer, and the glucose oxidase and the carrier protein are distributed in a substantially uniform manner throughout the layer. Preferably the layer is less than 2 microns in thickness. Surprisingly, sensors having these extremely thin analyte sensing layers have material properties that exceed those of sensors having thicker coatings including enhanced longevity, linearity, regularity as well as improved signal to noise ratios. While not being bound by a specific scientific theory, it is believed that sensors having extremely thin analyte sensing layers have surprisingly enhanced characteristics as compared to those of thicker layers because in thicker enzyme layers only a fraction of the reactive enzyme within the layer is able to access the analyte to be sensed. In sensors utilizing glucose oxidase, the thick coatings produced by electrodeposition may hinder the ability of hydrogen peroxide generated at the reactive interface of a thick enzyme layer to contact the sensor surface and thereby generate a signal.

As noted above, the enzyme and the second protein are typically treated to form a crosslinked matrix (e.g. by adding a cross-linking agent to the protein mixture). As is known in the art, crosslinking conditions may be manipulated to modulate factors such as the retrained biological activity of the enzyme, its mechanical and/or operational stability. Illustrative crosslinking procedures are described in U.S. Patent Application Serial Number 10/335,506 and PCT publication WO 03/035891. For example, an amine cross-linking reagent, such as, but not limited to, glutaraldehyde, can be added to the protein mixture. The addition of a cross-linking reagent to the protein mixture creates a protein paste. The concentration of the cross-linking reagent to be added may vary according to the concentration of the protein mixture. While glutaraldehyde is a preferred crosslinking reagent, other cross-linking reagents may also be used or may be used in place of gluturaldehyde, including, but not limited to, an amine reactive, homofunctional, cross-linking reagent such as Disuccinimidyl Suberate (DSS). Another example is 1-Ethyl-3 (3-Dimethylaminopropyl) Carbodiimide (EDC), which is a zero-length cross-linker. EDC forms an amide bond between carboxylic acid and amine groups. Other suitable cross-linkers also may be used, as will be evident to those skilled in the art.

The GOx and/or carrier protein concentration may vary for different embodiments of the invention. For example, the GOx concentration may be within the range of approximately 50 mg/ml (approximately 10,000 U/ml) to approximately 700 mg/ml (approximately 150,000 U/ml). Preferably the GOx concentration is about 115 mg/ml (approximately 22,000 U/ml). In such embodiments, the HSA concentration may vary between about 0.5%-30% (w/v), depending on the GOx concentration. Preferably the HSA concentration is about 1-10% w/v, and most preferably is about 5% w/v. In alternative embodiments of the invention, collagen or BSA or other structural proteins used in these contexts can be used instead of or in addition to HSA. Although GOx is discussed as a preferred enzyme in the analyte sensing layer, other proteins and/or enzymes may also be used or may be used in place of GOx, including, but not limited to glucose dehydrogenase or hexokinase, hexose oxidase, lactate oxidase, and the like. Other proteins and/or enzymes may also be used, as will be evident to those skilled in the art. Moreover, although HSA is employed in the example embodiment, other structural proteins, such as BSA, collagens or the like, could be used instead of or in addition to HSA.

For embodiments employing enzymes other than GOx, concentrations other than those discussed herein may be utilized. For example, depending on the enzyme employed, concentrations ranging from approximately 10% weight per weight to 70% weight per weight may be suitable. The concentration may be varied not only depending on the particular enzyme being employed, but also depending on the desired properties of the resulting protein matrix. For example, a certain concentration may be utilized if the protein matrix is to be used in a diagnostic capacity while a different concentration may be utilized if certain structural properties are desired. Those skilled in the art will understand that the concentration utilized may be varied through experimentation to determine which concentration (and of which enzyme or protein) may yield the desired result

As noted above, in preferred embodiments of the invention, the analyte sending layer includes a composition (e.g. glucose oxidase) capable of producing a signal (e.g. a change in oxygen and/or hydrogen peroxide concentrations) that can be sensed by the electrically conductive elements (e.g. electrodes which sense changes in oxygen and/or hydrogen peroxide concentrations). However, other useful analyte sensing layers can be formed from any composition that is capable of producing a detectable signal that can be sensed by the electrically conductive elements after interacting with a target analyte whose presence is to be detected. In preferred embodiments, the composition comprises an enzyme that modulates hydrogen peroxide concentrations upon reaction with an analyte to be sensed. Alternatively, the composition comprises an enzyme that modulates oxygen concentrations upon reaction with an analyte to be sensed. In this context, a wide variety of enzymes that either use or produce hydrogen peroxide and/or oxygen in a reaction with a physiological analyte are known in the art and these enzymes can be readily incorporated into the analyte sensing layer composition. A variety of other enzymes known in the art can produce and/or utilize compounds whose modulation can be detected by electrically conductive elements such as the electrodes that are incorporated into the preferred sensor designs described herein. Such enzymes include for example, enzymes specifically described in Table 1, pages 15-29 and/or Table 18, pages 111-112 of Protein Immobilization: Fundamentals and Applications (Bioprocess Technology, Vol 14) by Richard F. Taylor (Editor) Publisher: Marcel Dekker; (January 7, 1991).

Other useful analyte sensing layers can be formed to include antibodies whose interaction with a target analyte is capable of producing a detectable signal that can be sensed by the electrically conductive elements after interacting with the target analyte whose presence is to be detected. For example U.S. Patent No. 5,427,912 describes an antibody-based apparatus for electrochemically determining the concentration of an analyte in a sample. In this device, a mixture is formed which includes the sample to be tested, an enzyme-acceptor polypeptide, an enzyme-donor polypeptide linked to an analyte analog (enzyme-donor polypeptide conjugate), a labeled substrate, and an antibody specific for the analyte to be measured. The analyte and the enzyme-donor polypeptide conjugate competitively bind to the antibody. When the enzyme-donor polypeptide conjugate is not bound to antibody, it will spontaneously combine with the enzyme acceptor polypeptide to form an active enzyme complex. The active enzyme then hydrolyzes the labeled substrate, resulting in the generation of an electroactive label, which can then be oxidized at the surface of an electrode. A current resulting from the oxidation of the electroactive compound can be measured and correlated to the concentration of the analyte in the sample. U.S. Patent No. 5,149,630 describes an electrochemical specific binding assay of a ligand (e.g., antigen, hapten or antibody) wherein at least one of the components is enzyme-labelled, and which includes the step of determining the extent to which the transfer of electrons between the enzyme substrate and an electrode, associated with the substrate reaction, is perturbed by complex formation or by displacement of any ligand complex relative to unbound enzyme-labelled component The electron transfer is aided by electron-transfer mediators which can accept electrons from the enzyme and donate them to the electrode or vice versa (e.g. ferrocene) or by electron-transfer promoters which retain the enzyme in close proximity with the electrode without themselves taking up a formal charge. U.S. Patent No. 5,147,781 describes an assay for the determination of the enzyme lactate dehydrogenase-5 (LDH5) and to a biosensor for such quantitative determination. The assay is based on the interaction of this enzyme with the substrate lactic acid and nicotine-amine adenine dinucleotide (NAD) to yield pyruvic acid and the reduction product of NAD. Anti-LDH5 antibody is bound to a suitable glassy carbon electrode, this is contacted with the substrate containing LDH5, rinsed, inserted into a NAD solution, connected to an amperometic system, lactic acid is added and the current changes are measured, which are indicative of the quantity of LDH-5. U.S. Patent No. 6,410,251 describes an apparatus and method for detecting or assaying one constituting member in a specific binding pair, for example, the antigen in an antigen/antibody pair, by utilizing specific binding such as binding between an antigen and an antibody, together with redox reaction for detecting a label, wherein an oxygen micro-electrode with a sensing surface area is used. In addition, U.S. Patent No. 4,402,819 describes an antibody-selective potentiometric electrode for the quantitative determination of antibodies (as the analyte) in dilute liquid serum samples employing an insoluble membrane incorporating an antigen having bonded thereto an ion carrier effecting the permeability of preselected cations therein, which permeability is a function of specific antibody concentrations in analysis, and the corresponding method of analysis. For related disclosures, see also U.S. Patent Nos. 6,703,210, 5,981,203, 5,705,399 and 4,894,253.

In addition to enzymes and antibodies, other exemplary materials for use in the analyte sensing layers of the sensors disclosed herein include polymers that bind specific types of cells or cell components (e.g. polypeptides, carbohydrates and the like); single-strand DNA; antigens and the like. The detectable signal can be, for example, an optically detectable change, such as a color change or a visible accumulation of the desired analyte (e.g., cells). Sensing elements can also be formed from materials that are essentially non-reactive (i.e., controls). The foregoing alternative sensor elements are beneficially included, for example, in sensors for use in cell-sorting assays and assays for the presence of pathogenic organisms, such as viruses (HIV, hepatitis-C, etc.), bacteria, ptotozoa and the like.

Also contemplated are analyte sensors that measure an analyte that is present in the external environment and that can in itself produce a measurable change in current at an electrode. In sensors measuring such analytes, the analyte sensing layer can be optional.

### PROTEIN LAYER

The electrochemical sensors of the invention optionally include a protein layer disposed between the analyte sensing layer and the analyte modulating layer (see, e.g. element 116 in Figure 2). The term "protein layer" is used herein according to art accepted terminology and refers to layer containing a carrier protein or the like that is selected for compatibility with the analyte sensing layer and or the analyte modulating layer. In typical embodiments, the protein layer comprises an albumin such as human serum albumin. The HSA concentration may vary between about 0.5%-30% (w/v). Preferably the HSA concentration is about 1-10% w/v, and most preferably is about 5% w/v. In alternative embodiments of the invention, collagen or BSA or other structural proteins used in these contexts can be used instead of or in addition to HSA. This layer is typically crosslinked on the analyte sensing layer according to art accepted protocols.

### ADHESION PROMOTING LAYER

The electrochemical sensors of the invention can include one or more adhesion promoting (AP) layers (see, e.g. element 114 in Figure 2). The term "adhesion promoting layer" is used herein according to art accepted terminology and refers to a layer that includes materials selected for their ability to promote adhesion between adjoining layers in the sensor. Typically, the adhesion promoting layer is disposed between the analyte sensing layer and the analyte modulating layer. Preferably, the adhesion promoting layer is disposed between the optional protein layer and the analyte modulating layer. The adhesion promoter layer can be made from any one of a wide variety of materials known in the art to facilitate the bonding between such layers and can be applied by any one of a wide variety of methods known in the art. Preferably, the adhesion promoter layer comprises a silane compound such as γ-aminopropyltrimethoxysilane.

The use of silane coupling reagents, especially those of the formula R'Si(OR)₃ in which R' is typically an aliphatic group with a terminal amine and R is a lower alkyl group, to promote adhesion is known in the art (see, e.g. U.S. Patent No. 5,212,050 which is incorporated herein by reference). For example, chemically modified electrodes in which a silane such as γ-aminopropyltriethoxysilane and glutaraldehyde were used in a step-wise process to attach and to co-crosslink bovine serum albumin (BSA) and glucose oxidase (GOₓ) to the electrode surface are well known in the art (see, e.g. Yao, T. Analytica Chim. Acta 1983,148, 27-33).

In certain preferred embodiments of the invention, the adhesion promoting layer further comprises one or more compounds that can also be present in an adjacent layer such as the polydimethyl siloxane (PDMS) compounds that serves to limit the diffusion of analytes such as glucose through the analyte modulating layer. In illustrative embodiments the formulation comprises 0.5-20% PDMS, preferably 5-15% PDMS, and most preferably 10% PDMS. In certain embodiments of the invention, the adhesion promoting layer includes an agent selected for its ability to crosslink a siloxane moiety present in a proximal layer such as the analyte modulating layer. In closely related embodiments of the invention, the adhesion promoting layer includes an agent selected for its ability to crosslink an amine or carboxyl moiety of a protein present in a proximal layer such a the analyte sensing layer and/or the protein layer.

### ANALYTE MODULATING LAYER

The electrochemical sensors of the invention include an analyte modulating layer disposed on the sensor (see, e.g. element 112 in Figure 2). The term "analyte modulating layer" is used herein according to art accepted terminology and refers to a layer that typically forms a membrane on the sensor that operates to modulate the diffusion of one or more analytes, such as glucose, through the layer. In certain embodiments of the invention, the analyte modulating layer is an analyte limiting membrane which operates to prevent or restrict the diffusion of one or more analytes, such as glucose, through the layers. In other embodiments of the invention, the analyte modulating layer operates to facilitate the diffusion of one or more analytes, through the layers. Optionally such analyte modulating layers can be formed to prevent or restrict the diffusion of one type of molecule through the layer (e.g. glucose), while at the same time allowing or even facilitating the diffusion of other types of molecules through the layer (e.g. O₂).

With respect to glucose sensors, in known enzyme electrodes, glucose and oxygen from blood, as well as some interferants, such as ascorbic acid and uric acid diffuse through a primary membrane of the sensor. As the glucose, oxygen and interferants reach the analyte sensing layer, an enzyme, such as glucose oxidase, catalyzes the conversion of glucose to hydrogen peroxide and gluconolactone. The hydrogen peroxide may diffuse back through the analyte modulating layer, or it may diffuse to an electrode where it can be reacted to form oxygen and a proton to produce a current that is proportional to the glucose concentration. The sensor membrane assembly serves several functions, including selectively allowing the passage of glucose therethrough. In this context, a preferred analyte modulating layer is a semi-permeable membrane which permits passage of water, oxygen and at least one selective analyte and which has the ability to absorb water, the membrane having a water soluble, hydrophilic polymer.

A variety of illustrative analyte modulating compositions are known in the art and are described for example in U.S. Patent Nos. 6,319,540, 5,882,494, 5,786,439 5,777,060, 5,771,868 and 5,391,250, the disclosures of each being incorporated herein by reference. The hydrogels described therein are particularly useful with a variety of implantable devices for which it is advantageous to provide a surrounding water layer. In preferred embodiments of the invention, the analyte modulating composition includes PDMS. In certain embodiments of the invention, the analyte modulating layer includes an agent selected for its ability to crosslink a siloxane moiety present in a proximal layer. In closely related embodiments of the invention, the adhesion promoting layer includes an agent selected for its ability to crosslink an amine or carboxyl moiety of a protein present in a proximal layer.

### COVER LAYER

The electrochemical sensors of the invention may include one or more cover layers which are typically electrically insulating protective layers (see, e.g. element 106 in Figure 2). Typically, such cover layers are disposed on at least a portion of the analyte modulating layer. Acceptable polymer coatings for use as the insulating protective cover layer can include, but are not limited to, non-toxic biocompatible polymers such as silicone compounds, polyimides, biocompatible solder masks, epoxy acrylate copolymers, or the like. Further, these coatings can be photo-imageable to facilitate photolithographic forming of apertures through to the conductive layer. A typical cover layer comprises spun on silicone. As is known in the art, this layer can be a commercially available RTV (room temperature vulcanized) silicone composition. A typical chemistry in this context is polydimethyl siloxane (acetoxy based).

Various illustrative embodiments of the invention and their characteristics are discussed in detail in the following sections.

### C. ILLUSTRATIVE EMBODIMENTS OF ANALYTE SENSOR APPARATUS AND ASSOCIATED CHARACTERISTICS

The analyte sensor apparatus disclosed herein has a number of embodiments. A general embodiment of the invention is an analyte sensor apparatus for implantation within a mammal. While the analyte sensors are typically designed to be implantable within the body of a mammal, the sensor are not limited to any particular environment can instead be used in a wide variety of contexts, for example for the analysis of most liquid samples including biological fluids such as whole-blood, lymph, plasma, serum, saliva, urine, stool, perspiration, mucus, tears, cerebrospinal fluid, nasal secretion, cervical or vaginal secretion, semen, pleural fluid, amniotic fluid, peritoneal fluid, middle ear fluid, joint fluid, gastric aspirate or the like. In addition, solid or desiccated samples may be dissolved in an appropriate solvent to provide a liquid mixture suitable for analysis.

As noted above, the sensor embodiments disclosed herein can be used to sense analytes of interest in one of more physiological environments. In certain preferred embodiments for example, the sensor can be in direct contact with interstitial fluids as typically occurs with subcutaneous sensors. The sensors of the present invention may also be part of a skin surface system where interstitial glucose is extracted through the skin and brought into contact with the sensor (see, e.g. 6,155,992 and 6,706,159). In other embodiments, the sensor can be in contact with blood as typically occurs for example with intravenous sensors. The sensor embodiments of the invention further include those adapted for use in a variety of contexts. In certain preferred embodiments for example, the sensor can be designed for use in mobile contexts, such as those employed by ambulatory users. Alternatively, the sensor can be designed for use in stationary contexts such as those adapted for use in clinical settings. Such sensor embodiments include for example those used to monitor one or more analytes present in one or more physiological environments in a hospitalized patient.

Sensors of the invention can also be incorporated in to a wide variety of medical systems known in the art Sensors of the invention can be used for example in a closed loop infusion systems designed to control the rate that medication is infused into the body of a user. Such a closed loop infusion system can include a sensor and an associated meter which generates an input to a controller which in turn operates a delivery system (e.g. one that calculates a dose to be delivered by a medication infusion pump). In such contexts, the meter associated with the sensor may also transmit commands to, and be used to remotely control, the delivery system Preferably, the sensor is a subcutaneous sensor in contact with interstitial fluid to monitor the glucose concentration in the body of the user, and the liquid infused by the delivery system into the body of the user includes insulin. Illustrative systems are disclosed for example in U. S. Patent Nos. 6,558,351 and 6,551,276; PCT Application Nos. US99/21703 and US99/22993; as well as WO 2004/008956 and WO 2004/009161.

Certain embodiments of the invention measure peroxide and have the advantageous characteristic of being suited for implantation in a variety of sites in the mammal including regions of subcutaneous implantation and intravenous implantation as well as implantation into a variety of non-vascalar regions. A peroxide sensor design that allows implantation into non-vascular regions has advantages over certain sensor apparatus designs that measure oxygen due to the problems with oxygen noise that can occur in oxygen sensors implanted into non-vascular regions. For example in such implanted oxygen sensor apparatus designs, oxygen noise at the reference sensor can compromise the signal to noise ratio which consequently perturbs their ability to obtain stable glucose readings in this environment. The peroxide sensors of the invention therefore overcome the difficulties observed with such oxygen sensors in non-vascular regions.

Certain peroxide sensor embodiments of the invention further include advantageous long term or "permanent" sensors which are suitable for implantation in a mammal for a time period of greater than 30 days. In particular, as is known in the art (see, e.g. ISO 10993, Biological Evaluation of Medical Devices) medical devices such as the sensors described herein can be categorized into three groups based on implant duration: (1) "limited" (< 24 hours), (2) "Prolonged" (24 hours - 30 days), and (3) "Permanent" (> 30 days). In preferred embodiments of the invention, the design of the peroxide sensor of the invention allows for a "Permanent" implantation according to this categorization, i.e. > 30 days. In related embodiments of the invention, the highly stable design of the peroxide sensor of the invention allows for an implanted sensor to continue to function in this regard for 2, 3, 4, 5, 6 or 12 or more months.

In general, the analyte sensor apparatus structure comprises a base layer and a conductive layer disposed upon the base layer that includes one or more electrodes. For example, the conductive layer can include a working electrode, a reference electrode and/or a counter electrode. These electrodes can be spaced in proximity, or alternatively are spaced distally according to the preferred design. The sensor apparatus design is such that certain electrodes (e.g. the working electrode) can be exposed to the solution containing the analyte to be sensed (e.g. via an aperture) in the sensor apparatus. The sensor apparatus design is such that certain electrodes (e.g. the reference electrode) are not exposed to the solution containing the analyte to be sensed in the sensor apparatus.

Typically, the analyte sensor apparatus includes an analyte sensing layer disposed on the conductive layer, typically covering a portion or all of the working electrode. This analyte sensing layer detectably alters the electrical current at the working electrode in the conductive layer in the presence of an analyte to be sensed. As disclosed herein, this analyte sensing layer typically includes an enzyme or antibody molecule or the like that reacts with the analyte of interest in a manner that changes the concentrations of a molecule that can modulate the current at the working electrode (see e.g. oxygen and/or hydrogen peroxide as shown in the reaction scheme of FIG. 1). Illustrative analyte sensing layers comprise an enzyme such as glucose oxidase (e.g. for use in glucose sensors) or lactate oxidase (e.g. for use in lactate sensors). Typically, the analyte sensing layer further comprises a carrier protein in a substantially fixed ratio with the analyte sensing compound (e.g. the enzyme) and the analyte sensing compound and the carrier protein are distributed in a substantially uniform manner throughout the analyte sensing layer. Preferably the analyte sensing layer is very thin for example less than 1, 0.5, 0.25 or 0.1 microns in thickness. While not being bound by a specific scientific theory, it is believed that sensors having such thin analyte sensing layers have surprisingly enhanced characteristics as compared to the thicker layers that are typically generated by electrodeposition because electrodeposition produces 3-5 micron thick enzyme layers in which only a fraction of the reactive enzyme within the coating layer is able to access the analyte to be sensed. Such thicker glucose oxidase pellets that are produced by electrodeposition protocols are further observed to have a poor mechanical stability (e.g. a tendency to crack) and further take a longer time to prepare for actual use, typically taking weeks of testing before it is ready for implantation. As these problems are not observed with the thin layered enzyme coatings described herein, these thin coatings are preferred embodiments of the invention.

In sensors utilizing glucose oxidase for example, the thick coatings produced by electrodeposition may hinder the ability of hydrogen peroxide generated at the reactive interface of the 3-5 micron thick enzyme layer to contact the sensor surface and thereby generate a signal. In addition, hydrogen peroxide that is unable to reach a sensor surface due to such thick coatings can diffuse away from the sensor into the environment in which the sensor is placed, thereby decreasing the sensitivity and/or biocompatibility of such sensors. Moreover, while not being bound by a specific scientific theory, it is believed that sensors having such thin analyte sensing layers have unexpectedly advantageous properties that result from the fact that processes such as spin coating, or the like, allow for a precise control over the enzyme coating's ratio of glucose oxidase to albumin (which is used as a carrier protein to stabilize the glucose oxidase in the enzyme layer). Specifically, because glucose oxidase and albumin have different isoelectric points, electrodeposition processes may result in a surface coating in which an optimally determined ratio of enzyme to carrier protein is detrimentally altered in the electrodeposition process and further wherein the glucose oxidase and the carrier protein are not distributed in a substantially uniform manner throughout the disposed enzyme layer. In addition, sensors having such thin analyte sensing layers have unexpectedly faster response times. While not being bound by a specific scientific theory, it is believed that these surprising and advantageous properties result from the fact that thin enzyme layers allow a better access to the working electrode surface and may allow a greater proportion of the molecules that modulate current at the electrode to access the electrode surface. In this context, in certain sensor embodiments of the invention, an alteration in current in response to exposure to the analyte present in the body of the mammal can be detected via an amperometer within 15, 10, 5 or 2 minutes of the analyte contacting the analyte sensor.

Optionally, the analyte sensing layer has a protein layer disposed thereon and which it typically between this analyte sensing layer and the analyte modulating layer. A protein within the protein layer is an albumin selected from the group consisting of bovine serum albumin and human serum albumin. Typically this protein is crosslinked. Without being bound by a specific scientific theory, it is believed that this separate protein layer enhances sensor function provides surprising functional benefits by acting as a sort of capacitor that diminishes sensor noise (e.g. spurious background signals). For example, in the sensors of the invention, some amount of moisture may form under the analyte modulating membrane layer of the sensor, the layer which regulates the amount of analyte that can contact the enzyme of the analyte sensing layer. This moisture may create a compressible layer that shifts within the sensor as a patient using the sensor moves. Such shifting of layers within the sensor may alter the way that an analyte such as glucose moves through the analyte sensing layers in a manner that is independent of actual physiological analyte concentrations, thereby generating noise. In this context, the protein layer may act as a capacitor by protecting an enzyme such as GOₓ from contacting the moisture layer. This protein layer may confer a number of additional advantages such as promoting the adhesion between the analyte sensing layer and the analyte modulating membrane layer. Alternatively, the presence of this layer may result in a greater diffusion path for molecules such as hydrogen peroxide, thereby localizing it to the electrode sensing element and contributing to an enhanced sensor sensitivity.

Typically, the analyte sensing layer and/or the protein layer disposed on the analyte sensing layer has an adhesion promoting layer disposed thereon. Such adhesion promoting layers promote the adhesion between the analyte sensing layer and a proximal layer, typically an analyte modulating layer. This adhesion promoting layer preferably comprises a silane compound such as γ-aminopropyltrimethoxysilane which is selected for its ability to promote optimized adhesion between the various sensor layers and functions to stabilize the sensor. Interestingly sensors having such a silane containing adhesion promoting layers exhibit unexpected properties including an enhanced overall stability. In addition, silane containing adhesion promoting layers provide a number of advantageous characteristics in addition to an ability to enhancing sensor stability and can for example play a beneficial role in interference rejection as well as in controlling the mass transfer of one or more desired analytes.

In certain preferred embodiments of the invention, the adhesion promoting layer further comprises one or more compounds that can also be present in an adjacent layer such as the polydimethyl siloxane (PDMS) compounds that serves to limit the diffusion of analytes such as glucose through the analyte modulating layer. The addition of PDMS to the AP layer for example can be advantageous in contexts where it diminishes the possibility of holes or gaps occurring in the AP layer as the sensor is manufactured.

Typically the adhesion promoting layer has an analyte modulating layer disposed thereon which functions to modulate the diffusion of analytes therethrough. In one embodiment, the analyte modulating layer includes compositions (e.g. polymers and the like) which serves to enhance the diffusion of analytes (e.g. oxygen) through the sensor layers and consequently function to enrich analyte concentrations in the analyte sensing layer. Alternatively, the analyte modulating layer includes compositions which serve to limit the diffusion of analytes (e.g. glucose) through the sensor layers and consequently function to limit analyte concentrations in the analyte sensing layer. An illustrative example of this is a hydrophilic glucose limiting membrane (i.e. functions to limit the diffusion of glucose therethrough) comprising a polymer such as polydimethyl siloxane or the like.

Typically the analyte modulating layer further comprises one or more cover layers which are typically electrically insulating protective layers a cover layer disposed on at least a portion of the sensor apparatus (e.g. covering the analyte modulating layer). Acceptable polymer coatings for use as the insulating protective cover layer can include, but are not limited to, non-toxic biocompatible polymers such as silicone compounds, polyimides, biocompatible solder masks, epoxy acrylate copolymers, or the like. A preferred cover layer comprises spun on silicone. Typically the cover layer further includes an aperture that exposes at least a portion of a sensor layer (e.g. analyte modulating layer) to a solution comprising the analyte to be sensed.

The analyte sensors described herein can be polarized cathodically to detect for example, changes in current at the working cathode that result from the changes in oxygen concentration proximal to the working cathode that occur as glucose interacts with glucose oxidase as shown in FIG. 1. Alternatively, the analyte sensors described herein can be polarized anodically to detect for example, changes in current at the working anode that result from the changes in hydrogen peroxide concentration proximal to the working anode that occur as glucose interacts with glucose oxidase as shown in FIG. 1. In typical embodiments of the invention, the current at the working electrode(s) are compared to the current at a reference electrode(s) (a control), with the differences between these measurements providing a value that can then be correlated to the concentration of the analyte being measured. Analyte sensor designs that obtain a current value by obtaining a measurement from a comparison of the currents at these dual electrodes are commonly termed, for example, dual oxygen sensors.

In preferred embodiments of the invention, the analyte sensor apparatus is designed to function via anodic polarization such that the alteration in current is detected at the anodic working electrode in the conductive layer of the analyte sensor apparatus. Structural design features than can be associated with anodic polarization include designing an appropriate sensor configuration comprising a working electrode which is an anode, a counter electrode which is a cathode and a reference electrode and then selectively disposing the appropriate analyte sensing layer on the appropriate portion of the surface of the anode within this design configuration. Optionally this anodic polarization structural design includes anodes, cathodes and/or working electrodes having different sized surface areas. For example, this structural design includes features where the working electrode (anode) and/or the coated surface of the working electrode is larger than the counter electrode (cathode) and/or the coated surface of the counter electrode. In this context, the alteration in current that can be detected at the anodic working electrode is then correlated with the concentration of the analyte. In certain illustrative examples of this embodiment of the invention, the working electrode is measuring and utilizing hydrogen peroxide in the oxidation reaction (see e.g. FIG. 1), hydrogen peroxide that is produced by an enzyme such as glucose oxidase or lactate oxidase upon reaction with glucose or lactate respectively. Such embodiments of the invention relating to electrochemical glucose and/or lactate sensors having such hydrogen peroxide recycling capabilities are particularly preferred because the recycling of this molecule reduces the amount of hydrogen peroxide that can escape from the sensor into the environment in which it is placed. In this context, implantable sensors that are designed to reduce the release of tissue irritants such as hydrogen peroxide will have improved biocompatibility profiles. Moreover as it is observed that hydrogen peroxide can react with enzymes such as glucose oxidase and compromise their biological function, such sensors are preferred due to their avoidance of this phenomena. Optionally, the analyte modulating layer (e.g. a glucose limiting layer) can include compositions that serve to inhibit the diffusion of hydrogen peroxide out in to the environment in which the sensor is placed. Consequently, such embodiments of the invention improve the biocompatibility of sensors that incorporate enzymes that produce hydrogen peroxide by incorporating hydrogen peroxide recycling elements disclosed herein.

Certain embodiments of the analyte sensors of the invention that comprise a base layer, a conductive layer, an analyte sensing layer, an optional protein layer, an adhesion promoting layer, and analyte modulating layer and a cover layer exhibit a number of unexpected properties. For example, in sensors at are structured to function via anodic polarization versus those structured to function via cathodic polarization, differences in the electrochemical reactions in the analyte sensing layer as well as at the electrode surface generate and/or consume different chemical entities, thereby altering the chemical environment in which the various sensor elements function in different polarities. In this context the sensor structure disclosed herein provides a surprisingly versatile device that is shown to function with an unexpected degree of stability under a variety of different chemical and/or electrochemical conditions.

In certain embodiments of the invention disclosed herein (e.g., those having hydrogen peroxide recycling capabilities) the sensor layer has a plurality of electrodes including a working electrode (e.g. an anode) and a counter electrode (e.g. a cathode), both of which are coated with a analyte sensing layer comprising an enzyme such as glucose oxidase or lactate oxidase: Such sensor designs have surprising properties including an enhanced sensitivity. Without being bound by a specific theory, these properties may result from the enhanced oxidation of hydrogen peroxide at the surface of a working or a counter electrode which produces additional oxygen that can be utilized in the glucose sensing reaction (see, e.g., FIG. 1). Therefore this recycling effect may reduce the oxygen dependent limitations of certain sensor embodiments disclosed herein. Moreover, this design may result in a sensor having a working electrode that can readily reduce available hydrogen peroxide and consequently has a lower electrode potential. Sensors designed to function with lower electrode potentials are preferred embodiments of the invention because high electrode potentials in sensors of this type can result in a gas producing hydrolysis reaction which can destabilize the sensors (due to the disruption of sensor layers from gas bubbles produced by hydrolysis reactions). In addition, in sensor embodiments designed so that the counter electrode is coated with a very thin layer of an analyte sensing layer comprising an enzyme such as glucose oxidase or lactate oxidase, the hydrogen peroxide generated in the enzymatic reaction is very close to the reactive surface of the counter electrode. This can increase the overall efficiency of the sensor in a manner that allows for the production of compact sensor designs which include for example, counter electrodes with smaller reactive surfaces.

A specific illustrative example of an analyte sensor apparatus for implantation within a mammal is a peroxide sensor of the following design. A first layer of the peroxide sensor apparatus is a base layer, typically made from a ceramic such as alumina. A subsequent layer disposed upon the base layer is conductive layer including a plurality of electrodes including an anodic working electrode and a reference electrode. A subsequent layer disposed on the conductive layer is an analyte sensing layer that includes crosslinked glucose oxidase which senses glucose and consequently generates hydrogen peroxide as shown in Figure 1. In the presence of this hydrogen peroxide, the anodic working electrode experiences a measurable increase in current as the hydrogen peroxide generated contacts this anode in the conductive layer and is oxidized. The reference electrode serves as a control and is physically isolated from the working electrode and the hydrogen peroxide generated according to the reaction shown in Figure 1. This analyte sensing layer is preferably less than 1, 0.5, 0.25 or 0.1 microns in thickness and comprises a mixture of crosslinked human serum albumin in a substantially fixed ratio with the crosslinked glucose oxidase, with the glucose oxidase and the human serum albumin being distributed in a substantially uniform manner throughout the sensor layer. A subsequent layer disposed on the sensor layer is a protein layer comprising crosslinked human serum albumin. A subsequent layer disposed on the protein layer is an adhesion promoting layer which promotes the adhesion between the analyte sensing layer and/or the protein layer and an analyte modulating layer which disposed upon these layers. This adhesion promoting layer comprises a silane composition. A subsequent layer disposed on the adhesion promoting layer is the analyte modulating layer in the form of a hydrophilic glucose limiting membrane comprising PDMS which modulates the diffusion of glucose therethrough. A subsequent layer is a cover layer, typically composed of silicone, which is disposed on at least a portion of the analyte modulating layer, wherein the cover layer further includes an aperture that exposes at least a portion of the analyte modulating layer to the external glucose containing environment so that the glucose can access the analyte sensing layer on the working electrode. This peroxide sensor apparatus functions via anodic polarization such that the hydrogen peroxide signal that is generated by glucose diffusing through the analyte modulating layer and then reacts with the glucose oxidase in the analyte sensing layer creates a detectable change in the current at the anodic working electrode in the conductive layer of the sensor that can be measured by a amperometer. This change in the current at the anodic working electrode can then be correlated with the concentration of glucose in the external environment. Consequently, a sensor of this design can act as a peroxide based glucose sensor.

### D. PERMUTATIONS OF ANALYTE SENSOR APPARATUS AND ELEMENTS

As noted above, the invention disclosed herein includes a number of embodiments including sensors having very thin enzyme coatings. Such embodiments of the invention allow artisans to generate a variety of permutations of the analyte sensor apparatus disclosed herein. As noted above, illustrative general embodiments of the sensor disclosed herein include a base layer, a cover layer and at least one layer having a sensor element such as an electrode disposed between the base and cover layers. Typically, an exposed portion of one or more sensor elements (e.g., a working electrode, a counter electrode, reference electrode, etc.) is coated with a very thin layer of material having an appropriate electrode chemistry. For example, an enzyme such as lactate oxidase, glucose oxidase, glucose dehydrogenase or hexokinase, can be disposed on the exposed portion of the sensor element within an opening or aperture defined in the cover layer. FIG. 2 illustrates a cross-section of a typical sensor structure 100 of the present invention. The sensor is formed from a plurality of layers of various conductive and non-conductive constituents disposed on each other according to a method of the invention to produce a sensor structure 100.

As noted above, in the sensors of the invention, the various layers (e.g. the analyte sensing layer) of the sensors can have one or more bioactive and/or inert materials incorporated therein. The term "incorporated" as used herein is meant to describe any state or condition by which the material incorporated is held on the outer surface of or within a solid phase or supporting matrix of the layer. Thus, the material "incorporated" may, for example, be immobilized, physically entrapped, attached covalently to functional groups of the matrix layer(s). Furthermore, any process, reagents, additives, or molecular linker agents which promote the "incorporation" of said material may be employed if these additional steps or agents are not detrimental to, but are consistent with the objectives of the present invention. This definition applies, of course, to any of the embodiments of the present invention in which a bioactive molecule (e.g. an enzyme such as glucose oxidase) is "incorporated." For example, Certain layers of the sensors disclosed herein include a proteinaceous substance such as albumin which serves as a crosslinkable matrix. As used herein, a proteinaceous substance is meant to encompass substances which are generally derived from proteins whether the actual substance is a native protein, an inactivated protein, a denatured protein, a hydrolyzed species, or a derivatized product thereof. Examples of suitable proteinaceous materials include, but are not limited to enzymes such as glucose oxidase and lactate oxidase and the like, albumins (e.g. human serum albumin, bovine serum albumin etc.), caseins, gamma-globulins, collagens and collagen derived products (e.g., fish gelatin, fish glue, animal gelatin, and animal glue).

A preferred embodiment of the invention is shown in FIG. 2. This embodiment includes an electrically insulating base layer 102 to support the sensor 100. The electrically insulating layer base 102 can be made of a material such as a ceramic substrate, which may be self-supporting or further supported by another material as is known in the art. In an alternative embodiment, the electrically insulating layer 102 comprises a polyimide substrate, for example a polyimide tape, dispensed from a reel. Providing the layer 102 in this form can facilitate clean, high density mass production. Further, in some production processes using such a polyimide tape, sensors 100 can be produced on both sides of the tape.

Typical embodiments of the invention include an analyte sensing layer disposed on the base layer 102. In a preferred embodiment as shown in FIG. 2 the analyte sensing layer comprises a conductive layer 104 which is disposed on insulating base layer 102. Preferably the conductive layer 104 comprises one or more electrodes. The conductive layer 104 can be applied using many known techniques and materials as will be described hereafter, however, the electrical circuit of the sensor 100 is typically defined by etching the disposed conductive layer 104 into a desired pattern of conductive paths. A typical electrical circuit for the sensor 100 comprises two or more adjacent conductive paths with regions at a proximal end to form contact pads and regions at a distal end to form sensor electrodes. An electrically insulating protective cover layer 106 such as a polymer coating is typically disposed on portions of the conductive layer 104. Acceptable polymer coatings for use as the insulating protective layer 106 can include, but are not limited to, non-toxic biocompatible polymers such as polyimide, biocompatible solder masks, epoxy acrylate copolymers, or the like. Further, these coatings can be photo-imageable to facilitate photolithographic forming of apertures 108 through to the conductive layer 104.

In the sensors of the present invention, one or more exposed regions or apertures 108 can be made through the protective layer 106 to the conductive layer 104 to define the contact pads and electrodes of the sensor 100. In addition to photolithographic development, the apertures 108 can be formed by a number of techniques, including laser ablation, chemical milling or etching or the like. A secondary photoresist can also be applied to the cover layer 106 to define the regions of the protective layer to be removed to form the apertures 108. An operating sensor 100 typically includes a plurality of electrodes such as a working electrode and a counter electrode electrically isolated from each other, however typically situated in close proximity to one another. Other embodiments may also include a reference electrode. Still other embodiments may utilize an separate reference element not formed on the sensor. The exposed electrodes and/or contact pads can also undergo secondary processing through the apertures 108, such as additional plating processing, to prepare the surfaces and/or strengthen the conductive regions.

A analyte sensing layer 110 is typically disposed on one or more of the exposed electrodes of the conductive layer 104 through the apertures 108. Preferably, the analyte sensing layer 110 is a sensor chemistry layer and most preferably an enzyme layer. Preferably, the analyte sensing layer 110 comprises the enzyme glucose oxidase or the enzyme lactate oxidase. In such embodiments, the analyte sensing layer 110 reacts with glucose to produce hydrogen peroxide which modulates a current to the electrode which can be monitored to measure an amount of glucose present. The sensor chemistry layer 110 can be applied over portions of the conductive layer or over the entire region of the conductive layer. Preferably the sensor chemistry layer 110 is disposed on portions of a working electrode and a counter electrode that comprise a conductive layer. Preferred methods for generating the thin sensor chemistry layer 110 include spin coating processes, dip and dry processes, low shear spraying processes, ink-jet printing processes, silk screen processes and the like. Most preferably the thin sensor chemistry layer 110 is applied using a spin coating process.

The analyte sensing layer 110 is typically coated with one or more coating layers. In preferred embodiments of the invention, one such coating layer includes a membrane which can regulate the amount of analyte that can contact an enzyme of the analyte sensing layer. For example, a coating layer can comprise an analyte modulating membrane layer such as a glucose limiting membrane which regulates the amount of glucose that contacts the glucose oxidase enzyme layer on an electrode. Such glucose limiting membranes can be made from a wide variety of materials known to be suitable for such purposes, e.g., silicone, polyurethane, polyurea cellulose acetate, Nafion, polyester sulfonic acid (Kodak AQ), hydrogels or any other membrane known to those skilled in the art.

In preferred embodiments of the invention, a coating layer is a glucose limiting membrane layer 112 which is disposed above the sensor chemistry layer 110 to regulate glucose contact with the sensor chemistry layer 110. In some embodiments of the invention, an adhesion promoter layer 114 is disposed between the membrane layer 112 and the sensor chemistry layer 110 as shown in FIG. 2 in order to facilitate their contact and/or adhesion. The adhesion promoter layer 114 can be made from any one of a wide variety of materials known in the art to facilitate the bonding between such layers. Preferably, the adhesion promoter layer 114 comprises a silane compound. In alternative embodiments, protein or like molecules in the sensor chemistry layer 110 can be sufficiently crosslinked or otherwise prepared to allow the membrane layer 112 to be disposed in direct contact with the sensor chemistry layer 110 in the absence of an adhesion promoter layer 114.

As noted above, embodiments of the present invention can include one or more functional coating layers. As used herein, the term "functional coating layer" denotes a layer that coats at least a portion of at least one surface of a sensor, more preferably substantially all of a surface of the sensor, and that is capable of interacting with one or more analytes, such as chemical compounds, cells and fragments thereof, etc., in the environment in which the sensor is disposed. Non-limiting examples of functional coating layers include sensor chemistry layers (e.g., enzyme layers), analyte limiting layers, biocompatible layers; layers that increase the slipperiness of the sensor; layers that promote cellular attachment to the sensor; layers that reduce cellular attachment to the sensor, and the like. Typically analyte modulating layers operate to prevent or restrict the diffusion of one or more analytes, such as glucose, through the layers. Optionally such layers can be formed to prevent or restrict the diffusion of one type of molecule through the layer (e.g. glucose), while at the same time allowing or even facilitating the diffusion of other types of molecules through the layer (e.g. O₂). An illustrative functional coating layer is a hydrogel such as those disclosed in U.S. Patent Nos. 5,786,439 and 5,391,250. The hydrogels described therein are particularly useful with a variety of implantable devices for which it is advantageous to provide a surrounding water layer.

The sensor embodiments disclosed herein can include layers having UV-absorbing polymers. In accordance with one aspect of the present invention, there is provided a sensor including at least one functional coating layer including a UV-absorbing polymer. In preferred embodiments, the UV-absorbing polymer is a polyurethane, a polyurea or a polyurethane/polyurea copolymer. More preferably, the selected UV-absorbing polymer is formed from a reaction mixture including a diisocyanate, at least one diol, diamine or mixture thereof, and a polyfunctional UV-absorbing monomer.

UV-absorbing polymers are used with advantage in a variety of sensor fabrication methods, such as those described in U.S. Pat. No. 5,390,671, to Lord et aL, entitled "Transcutaneous Sensor Insertion Set"; No. 5,165,407, to Wilson et aL, entitled "implantable Glucose Sensor"; and U.S. Pat. No. 4,890,620, to Gough, entitled "Two-Dimensional Diffusion Glucose Substrate Sensing Electrode". However, any sensor production method which includes the step of forming a UV-absorbing polymer layer above or below a sensor element is considered to be within the scope of the present invention. In particular, the inventive methods are not limited to thin-film fabrication methods, and can work with other sensor fabrication methods that utilize UV-laser cutting. Embodiments can work with thick-film, planar or cylindrical sensors and the like, and other sensor shapes requiring laser cutting.

As disclosed herein, the sensors of the present invention are particularly designed for use as subcutaneous or transcutaneous glucose sensors for monitoring blood glucose levels in a diabetic patient Typically each sensor comprises a plurality of sensor element, for example electrically conductive elements such as elongated thin film conductors, formed between an underlying insulative thin film base layer and an overlying insulative thin film cover layer.

If desired, a plurality of different sensor elements can be included in a single sensor. For example, both conductive and reactive sensor elements can be combined in one sensor, optionally with each sensor element being disposed on a different portion of the base layer. One or more control elements can also be provided. In such embodiments, the sensor can have defined in its cover layer a plurality of openings or apertures. One or more openings can also be defined in the cover layer directly over a portion of the base layer, in order to provide for interaction of the base layer with one or more analytes in the environment in which the sensor is disposed. The base and cover layers can be comprised of a variety of materials, typically polymers. In more specific embodiments the base and cover layers are comprised of an insulative material such as a polyimide. Openings are typically formed in the cover layer to expose distal end electrodes and proximal end contact pads. In a glucose monitoring application, for example, the sensor can be placed transcutaneously so that the distal end electrodes are in contact with patient blood or extracellular fluid, and the contact pads are disposed externally for convenient connection to a monitoring device.

The sensors of the invention can have any desired configuration, for example planar or cylindrical. The base layer 102 can be self-supportive, such as a rigid polymeric layer, or non-self supportive, such as a flexible film. The latter embodiment is desirable in that it permits continuous manufacture of sensors using, for example, a roll of a polymeric film which is continuously unwound and upon which sensor elements and coating layers are continuously applied.

A general embodiment of the invention is a sensor designed for implantation within a body that comprises a base layer, an analyte sensing layer disposed upon the base layer which includes a plurality of sensor elements, an enzyme layer (preferably less than 2 microns in thickness) disposed upon the analyte sensing layer which coats all of the plurality of sensing elements on the conductive layer, and one or more coating layers. Typically the enzyme layer comprises glucose oxidase, preferably in a substantially fixed ratio with a carrier protein. In a specific embodiment, the glucose oxidase and the carrier protein are distributed in a substantially uniform manner throughout the disposed enzyme layer. Typically the carrier protein comprises albumin, preferably in an amount of about 5% by weight. As used herein, "albumin" refers to those albumin proteins typically used by artisans to stabilize polypeptide compositions such as human serum albumin, bovine serum albumin and the like. In highly preferred embodiments of the invention, a coating layer is an analyte contacting layer which is disposed on the sensor so as to regulate the amount of analyte that can contact the enzyme layer. In further highly preferred embodiments, the sensor includes an adhesion promoter layer disposed between the enzyme layer and the analyte contacting layer and the enzyme layer is less than 1, 0.5, 0.25 or 0.1 microns in thickness.

One aspect of the present invention involves processes for making sensors having improved electrode chemistry coatings (e.g., enzyme coatings of less than 2 microns in thickness) with enhanced material properties. Methods for producing the extremely thin enzyme coatings of the invention include spin coating processes, dip and dry processes, low shear spraying processes, ink-jet printing processes, silk screen processes and the like. Typically, such coatings are vapor crosslinked subsequent to their application. Surprisingly, sensors produced by these processes have material properties that exceed those of sensors having coatings produced by electrodeposition including enhanced longevity, linearity, regularity as well as improved signal to noise ratios. In addition, certain sensor embodiments of the invention that utilize glucose oxidase coatings formed by such processes are designed to recycle hydrogen peroxide and improve the biocompatibility profiles of such sensors.

In this context, a preferred embodiment of the invention is a method of making a less than about 2 micron coating of stabilized glucose oxidase on the surface of a matrix such as an electrode comprising combining glucose oxidase with albumin in a fixed ratio (one that is typically optimized for glucose oxidase stabilizing properties) and applying the glucose oxidase and albumin mixture to the surface of the matrix by a process selected from the group consisting of a spin coating process, a dip and dry process, a microdeposition process, a jet printer deposition process, a screen printing process or a doctor blading process. Preferably the stabilized glucose oxidase coating is applied to the surface of an electrode by a spin coating process. In highly preferred embodiments, the glucose oxidase/albumin is prepared in a physiological solution (e.g., phosphate buffered saline at neutral pH) with the albumin being present in an amount of about 5% albumin by weight. Optionally the stabilized glucose oxidase layer that is formed on the conductive layer is less than 2, 1, 0.5, 0.25 or 0.1 microns in thickness. A closely related embodiment of the invention is a stabilized glucose oxidase layer for coating the surface of an electrode wherein the glucose oxidase is mixed with a carrier protein in a fixed ratio within the layer, the glucose oxidase and the carrier protein are distributed in a substantially uniform manner throughout the layer. Preferably the layer is less than 2 microns in thickness.

A related embodiment of the invention is an electrochemical analyte sensor which includes a base layer, a conductive layer disposed upon the base layer that includes at least one working electrode and at least one counter electrode, an analyte sensing layer disposed upon the conductive layer, wherein the analyte sensing layer is less than 2 microns in thickness; and an analyte modulating layer that regulates the amount of analyte that contacts the enzyme layer, typically by limiting the amount of analyte that can diffuse through the layer and contact the analyte sensing layer. In an optional embodiment of the invention, the working electrode and/or the coated surface of the working electrode is larger than counter electrode and/or the coated surface of the counter electrode. In preferred embodiments, the enzyme layer comprises glucose oxidase stabilized by coating it on the working electrode and the counter electrode in combination with a carrier protein in a fixed ratio. In a highly preferred embodiment, this glucose oxidase enzyme layer substantially covers the conductive layer. Embodiments where the glucose oxidase enzyme layer is disposed in a uniform coating over the whole conductive layer are preferred because they may avoid problems associated with sensors having multiple different coatings on a single layer such as the selective delamination of different coatings having different material properties. Preferably, the sensor includes an adhesion promoting layer disposed between the enzyme layer and the analyte modulating layer.

A related embodiment of the invention is an electrochemical analyte sensor which includes a base layer, a conductive layer disposed upon the base layer that includes at least one working electrode, at least one reference electrode and at least one counter electrode, an enzyme layer disposed upon the conductive layer, and an analyte modulating cover layer that regulates the amount of analyte that contacts the enzyme layer. In preferred embodiments, the enzyme layer is less than 2 microns in thickness and is coated on at least a portion of the working electrode, the reference electrode and the counter electrode. In a highly preferred embodiment, the enzyme layer substantially covers the working electrode, the reference electrode and the counter electrode. Optionally, the enzyme layer comprises glucose oxidase in combination with a carrier protein (e.g. albumin) in a fixed ratio. Typically, the sensor includes an adhesion promoting layer disposed between the enzyme layer and the analyte modulating layer.

Yet another embodiment of the invention comprises a glucose sensor for implantation within a body which includes a base layer, a conductive layer disposed upon the base layer, an analyte sensing layer comprising glucose oxidase disposed upon the conductive layer, wherein the glucose oxidase is stabilized by combining it with albumin in a defined ratio and further wherein the glucose oxidase and the albumin are distributed in a substantially uniform manner throughout the disposed layer, and a glucose limiting layer that regulates the amount of glucose that diffuses through the glucose limiting layer and contacts the glucose oxidase layer. In preferred embodiments, the conductive layer includes a plurality of sensor elements including at least one working electrode and at least one counter electrode. In such sensor embodiments, the analyte sensing layer comprising glucose oxidase is preferably less than 2, 1, 0.5, 0.25 or 0.1 microns in thickness and the albumin in the layer is present in an amount of about 5% albumin by weight. Preferably the sensor includes an adhesion promoting layer disposed between the analyte sensing layer comprising glucose oxidase and the glucose limiting layer.

### E. ANALYTE SENSOR APPARATUS CONFIGURATIONS

In a clinical setting, accurate and relatively fast determinations of analytes such as glucose and/or lactate levels can be determined from blood samples utilizing electrochemical sensors. Conventional sensors are fabricated to be large, comprising many serviceable parts, or small, planar-type sensors which may be more convenient in many circumstances. The term "planar" as used herein refers to the well-known procedure of fabricating a substantially planar structure comprising layers of relatively thin materials, for example, using the well-known thick or thin-film techniques. See, for example, Liu et al., U.S. Pat. No. 4,571,292, and Papadakis et aL, U.S. Pat. No. 4,536,274. As noted below, embodiments of the invention disclosed herein have a wider range of geometrical configurations (e.g. planar) than existing sensors in the art. In addition, certain embodiments of the invention include one or more of the sensors disclosed herein coupled to another apparatus such as a medication infusion pump.

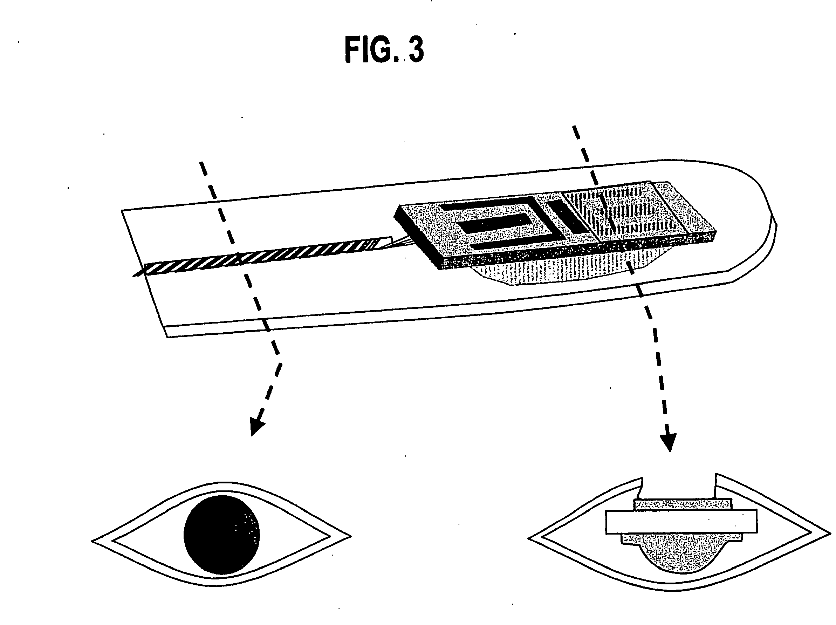

Figure 2 provides a diagrammatic view of a typical analyte sensor configuration of the current invention. Figure 3 provides an overview (upper) and cross sectional views (lower) of a relatively flat "ribbon" type configuration that can be made with the analyte sensor apparatus. Such "ribbon" type configurations illustrate an advantage of the sensors disclosed herein that arises due to the spin coating of sensing enzymes such as glucose oxidase, a manufacturing step that produces extremely thin enzyme coatings that allow for the design and production of highly flexible sensor geometries. Such thin enzyme coated sensors provide further advantages such as allowing for a smaller sensor area while maintaining sensor sensitivity, a highly desirable feature for implantable devices (e.g. smaller devices are easier to implant). Consequently, sensor embodiments of the invention that utilize very thin analyte sensing layers that can be formed by processes such as spin coating can have a wider range of geometrical configurations (e.g. planar) than those sensors that utilizes enzyme layers formed via processes such as electrodeposition.

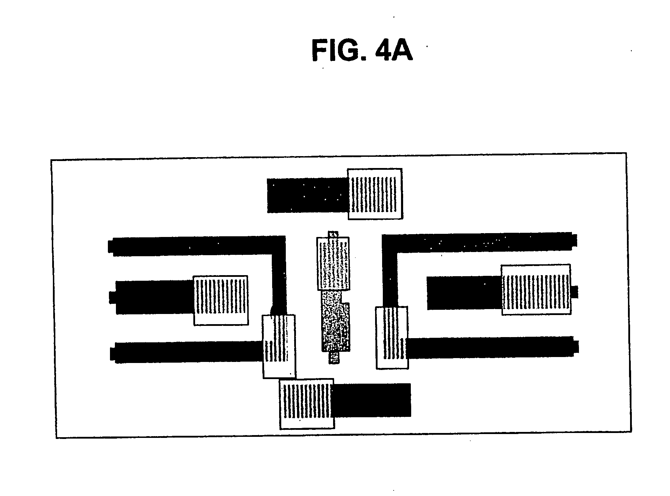

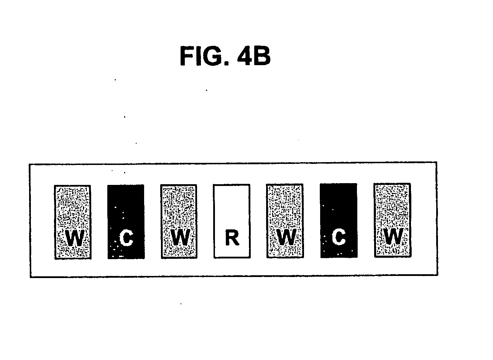

Figures 4A and 4B illustrate various sensor configurations that include multiple conductive elements such as multiple working, counter and reference electrodes. Advantages of such configurations include increased surface are which provides for greater sensor sensitivity. For example in the sensor configuration shown in Figure 4B, this pattern (including seven vias) introduces a third working sensor. One obvious advantage of such a configuration is signal averaging of three sensors which increases sensor accuracy. Other advantages include the ability to measure multiple analytes. In particular, analyte sensor configurations that include electrodes in this arrangement (e.g. multiple working, counter and reference electrodes) and be incorporated into multiple analyte sensors. The measurement of multiple analytes such as oxygen, hydrogen peroxide, glucose, lactate, potassium, calcium, and any other physiologically relevant substance/analyte provides a number of advantages, for example the ability of such sensors to provide a linear response as well as ease in calibration and/or recalibration.

An exemplary multiple sensor device comprises a single device having a first sensor which is polarized cathodically and designed to measure the changes in oxygen concentration that occur at the working electrode (a cathode) as a result of glucose interacting with glucose oxidase; and a second sensor which is polarized anodically and designed to measure changes in hydrogen peroxide concentration that occurs at the working electrode (an anode) as a result of glucose coming form the external environment and interacting with glucose oxidase. As is known in the art, in such designs, the first oxygen sensor will typically experience a decrease in current at the working electrode as oxygen contacts the sensor while the second hydrogen peroxide sensor will typically experience an increase in current at the working electrode as the hydrogen peroxide generated as shown in Figure 1 contacts the sensor. In addition, as is known in the art, an observation of the change in current that occurs at the working electrodes as compared to the reference electrodes in the respective sensor systems correlates to the change in concentration of the oxygen and hydrogen peroxide molecules which can then be correlated to the concentration of the glucose in the external environment (e.g. the body of the mammal).

Figure 5A provides an illustration of how the analyte sensors of the invention can be coupled with other medical devices such as medication infusion pumps. Figures 5B provides an illustration of a variation of this scheme where replaceable analyte sensors of the invention can be coupled with other medical devices such as medication infusion pumps, for example by the use of a port couple to the medical device (e.g. a subcutaneous port with a locking electrical connection).

### II. ILLUSTRATIVE METHODS AND MATERIALS FOR MAKING ANALYTE SENSOR APPARATUS OF THE INVENTION

A number of articles, U.S. patents and patent application describe the state of the art with the common methods and materials disclosed herein and further describe various elements (and methods for their manufacture) that can be used in the sensor designs disclosed herein. These include for example, U.S. Patent Nos. 6,413,393; 6,368,274; 5,786,439; 5,777,060; 5,391,250; 5,390,671; 5,165,407, 4,890,620, 5,390,671, 5,390,691, 5,391,250, 5,482,473, 5,299,571, 5,568,806; United States Patent Application 20020090738; as well as PCT International Publication Numbers WO 01/58348, WO 03/034902, WO 03/035117, WO 03/035891, WO 03/023388, WO 03/022128, WO 03/022352, WO 03/023708, WO 03/036255, WO03/036310 and WO 03/074107.

Typical sensors for monitoring glucose concentration of diabetics are further described in Shichiri, et al.,: "In Vivo Characteristics of Needle-Type Glucose Sensor-Measurements of Subcutaneous Glucose Concentrations in Human Volunteers," Horm. Metab. Res., Suppl. Ser. 20:17-20 (1988); Bruckel, et aL,: "In Vivo Measurement of Subcutaneous Glucose Concentrations with an Enzymatic Glucose Sensor and a Wick Method," Klin. Wochenschr. 67:491-495 (1989); and Pickup, et al.,: "In Vivo Molecular Sensing in Diabetes Mellitus: An Implantable Glucose Sensor with Direct Electron Transfer," Diabetologia 32:213-217 (1989). Other sensors are described in, for example Reach, et aL, in ADVANCES IN IMPLANTABLE DEVICES, A. Turner (ed.), JAI Press, London, Chap. 1, (1993).

### A. GENERAL METHODS FOR MAKING ANALYTE SENSORS

A typical embodiment of the invention disclosed herein is a method of making a sensor apparatus for implantation within a mammal comprising the steps of: providing a base layer; forming a conductive layer on the base layer, wherein the conductive layer includes an electrode (and typically a working electrode, a reference electrode and a counter electrode); forming an analyte sensing layer on the conductive layer, wherein the analyte sensing layer includes a composition that can alter the electrical current at the electrode in the conductive layer in the presence of an analyte; optionally forming a protein layer on the analyte sensing layer; forming an adhesion promoting layer on the analyte sensing layer or the optional protein layer; forming an analyte modulating layer disposed on the adhesion promoting layer, wherein the analyte modulating layer includes a composition that modulates the diffusion of the analyte therethrough; and forming a cover layer disposed on at least a portion of the analyte modulating layer, wherein the cover layer further includes an aperture over at least a portion of the analyte modulating layer. In certain embodiments of these methods, the analyte sensor apparatus is formed in a planar geometric configuration

As disclosed herein, the various layers of the sensor can be manufactured to exhibit a variety of different characteristics which can be manipulated according to the preferred design of the sensor. For example, the adhesion promoting layer includes a compound selected for its ability to stabilize the overall sensor structure, preferably a silane composition. In preferred embodiments of the invention, the analyte sensing layer is formed by a spin coating process and is of a thickness selected from the group consisting of less than 1, 0.5, 0.25 and 0.1 microns in height.

Preferably a method of making the sensor includes the step of forming a protein layer on the analyte sensing layer, wherein a protein within the protein layer is an albumin selected from the group consisting of bovine serum albumin and human serum albumin. Preferably a method of making the sensor includes the step of forming an analyte sensing layer that comprises an enzyme composition selected from the group consisting of glucose oxidase, glucose dehydrogenase, lactate oxidase, hexokinase and lactose dehydrogenase. In such methods, the analyte sensing layer preferably comprises a carrier protein composition in a substantially fixed ratio with the enzyme and the enzyme and the carrier protein are distributed in a substantially uniform manner throughout the analyte sensing layer.

### B. TYPICAL PROTOCOLS AND MATERIALS USEFUL IN THE MANUFACTURE OF ANALYTE SENSORS

The disclosure provided herein includes sensors and sensor designs that can be generated using combinations of various well known techniques. The disclosure further provides methods for applying very thin enzyme coatings to these types of sensors as well as sensors produced by such processes. In this context, preferred embodiments of the invention include methods for making such sensors on a substrate according to art accepted processes. In certain embodiments, the substrate comprises a rigid and flat structure suitable for use in photolithographic mask and etch processes. In this regard, the substrate typically defines an upper surface having a high degree of uniform flatness. A polished glass plate may be used to define the smooth upper surface. Alternative substrate materials include, for example, stainless steel, aluminum, and plastic materials such as delrin, etc. In other embodiments, the substrate is non-rigid and can be another layer of film or insulation that is used as a substrate, for example plastics such as polyimides and the like.

An initial step in the methods of the invention typically includes the formation of a base layer of the sensor. The base layer can be disposed on the substrate by any desired means, for example by controlled spin coating. In addition, an adhesive may be used if there is not sufficient adhesion between the substrate layer and the base layer. A base layer of insulative material is formed on the substrate, typically by applying the base layer material onto the substrate in liquid form and thereafter spinning the substrate to yield the base layer of thin, substantially uniform thickness. These steps are repeated to build up the base layer of sufficient thickness, followed by a sequence of photolithographic and/or chemical mask and etch steps to form the conductors discussed below. In a preferred form, the base layer comprises a thin film sheet of insulative material, such as ceramic or polyimide substrate. The base layer can comprise an alumina substrate, a polyimide substrate, a glass sheet, controlled pore glass, or a planarized plastic liquid crystal polymer. The base layer may be derived from any material containing one or more of a variety of elements including, but not limited to, carbon, nitrogen, oxygen, silicon, sapphire, diamond, aluminum, copper, gallium, arsenic, lanthanum, neodymium, strontium, titanium, yttrium, or combinations thereof. Additionally, the substrate may be coated onto a solid support by a variety of methods well-known in the art including chemical vapor deposition, physical vapor deposition, or spin-coating with materials such as spin glasses, chalcogenides, graphite, silicon dioxide, organic synthetic polymers, and the like.

The methods of the invention further include the generation of a conductive layer having one or more sensing elements. Typically these sensing elements are electrodes that are formed by one of the variety of methods known in the art such as photoresist, etching and rinsing to define the geometry of the active electrodes. The electrodes can then be made electrochemically active, for example by electrodeposition of Pt black for the working and counter electrode, and silver followed by silver chloride on the reference electrode. A sensor layer such as a sensor chemistry enzyme layer can then be disposed on the sensing layer by electrochemical deposition or a method other than electrochemical deposition such a spin coating, followed by vapor crosslinking, for example with a dialdehyde (glutaraldehyde) or a carbodi-imide.

Electrodes of the invention can be formed from a wide variety of materials known in the art. For example, the electrode may be made of a noble late transition metals. Metals such as gold, platinum, silver, rhodium, iridium, ruthenium, palladium, or osmium can be suitable in various embodiments of the invention. Other compositions such as carbon or mercury can also be useful in certain sensor embodiments. Of these metals, silver, gold, or platinum is typically used as a reference electrode metal. A silver electrode which is subsequently chloridized typically used as the reference electrode. These metals can be deposited by any means known in the art, including the plasma deposition method cited, supra, or by an electroless method which may involve the deposition of a metal onto a previously metallized region when the substrate is dipped into a solution containing a metal salt and a reducing agent. The electroless method proceeds as the reducing agent donates electrons to the conductive (metallized) surface with the concomitant reduction of the metal salt at the conductive surface. The result is a layer of adsorbed metal. (For additional discussions on electroless methods, see: Wise, E. M. Palladium: Recovery, Properties, and Uses, Academic Press, New York, New York (1988); Wong, K. et al. Plating and Surface Finishing 1988, 75, 70-76; Matsuoka, M. et al. Ibid. 1988, 75, 102-106; and Pearlstein, F. "Electroless Plating," Modern Electroplating, Lowenheim, F. A., Ed., Wiley, New York, N.Y. (1974), Chapter 31.). Such a metal deposition process must yield a structure with good metal to metal adhesion and minimal surface contamination, however, to provide a catalytic metal electrode surface with a high density of active sites. Such a high density of active sites is a property necessary for the efficient redox conversion of an electroactive species such as hydrogen peroxide.

In an exemplary embodiment of the invention, the base layer is initially coated with a thin film conductive layer by electrode deposition, surface sputtering, or other suitable process step. In the preferred form, this conductive layer may be provided as a plurality of thin film conductive layers, such as an initial chrome-based layer suitable for chemical adhesion to a polyimide base layer followed by subsequent formation of thin film gold-based and chrome-based layers in sequence. In alternative embodiments, other electrode layer conformations or materials can be used. The conductive layer is then covered, in accordance with conventional photolithographic techniques, with a selected photoresist coating, and a contact mask can be applied over the photoresist coating for suitable photoimaging. The contact mask typically includes one or more conductor trace patterns for appropriate exposure of the photoresist coating, followed by an etch step resulting in a plurality of conductive sensor traces remaining on the base layer. In an illustrative sensor construction designed for use as a subcutaneous glucose sensor, each sensor trace can include three parallel sensor elements corresponding with three separate electrodes such as a working electrode, a counter electrode and a reference electrode.

Portions of the conductive sensor layers are typically covered by a insulative cover layer, preferably of a material such as a silicon polymer and/or a polyimide. The insulative cover layer can be applied in any desired manner. In an exemplary procedure, the insulative cover layer is applied in a liquid layer over the sensor traces, after which the substrate is spun to distribute the liquid material as a thin film overlying the sensor traces and extending beyond the marginal edges of the sensor traces in sealed contact with the base layer. This liquid material can then be subjected to one or more suitable radiation and/or chemical and/or heat curing steps as are known in the art. In alternative embodiments, the liquid material can be applied using spray techniques or any other desired means of application. Various insulative layer materials may be used such as photoimagable epoxyacrylate, with a preferred material comprising a photoimagable polyimide available from OCG, Inc. of West Paterson, N.J., under the product number 7020.

As noted above, appropriate electrode chemistries defining the distal end electrodes can be applied to the sensor tips, optionally subsequent to exposure of the sensor tips through the openings. In an illustrative sensor embodiment having three electrodes for use as a glucose sensor, an enzyme (preferably glucose oxidase) is provided within one of the openings, thus coating one of the sensor tips to define a working electrode. One or both of the other electrodes can be provided with the same coating as the working electrode. Alternatively, the other two electrodes can be provided with other suitable chemistries, such as other enzymes, left uncoated, or provided with chemistries to define a reference electrode and a counter electrode for the electrochemical sensor.

A significant aspect of the present invention involves processes for making sensors having extremely thin coatings for electrode chemistries (e.g., enzyme coatings of less than 2 microns in thickness) with enhanced material properties. Methods for producing the extremely thin enzyme coatings of the invention include spin coating processes, dip and dry processes, low shear spraying processes, ink-jet printing processes, silk screen processes and the like. As artisans can readily determine the thickness of an enzyme coat applied by process of the art, they can readily identify those methods capable of generating the extremely thin coatings of the invention. Typically, such coatings are vapor crosslinked subsequent to their application. Surprisingly, sensors produced by these processes have material properties that exceed those of sensors having coatings produced by electrodeposition including enhanced longevity, linearity, regularity as well as improved signal to noise ratios. In addition, embodiments of the invention that utilize glucose oxidase coatings formed by such processes are designed to recycle hydrogen peroxide and improve the biocompatibility profiles of such sensors.

While not being bound by a specific scientific theory, it is believed that the surprising properties of sensors produced by such processes have enhanced characteristics as compared to those generated by electrodeposition because electrodeposition produces 3-5 micron thick enzyme layers in which only a fraction of the reactive enzyme is able to access the analyte to be sensed. Moreover, in sensors utilizing glucose oxidase, the thick coatings produced by electrodeposition may hinder the ability of hydrogen peroxide generated at the reactive interface to reach the sensor surface and thereby generate a signal. Moreover, hydrogen peroxide that is unable to reach a sensor surface due to such thick coatings typically diffuses away from the sensor into the environment in which the sensor is placed, thereby decreasing the biocompatibility of such sensors. In addition, as glucose oxidase and albumin have different isoelectric points, electrodeposition processes can result in a surface coating in which an optimally determined ratio of enzyme to carrier protein is detrimentally altered and further wherein the glucose oxidase and the carrier protein are not distributed in a substantially uniform manner throughout the disposed enzyme layer. The thin coating processes utilized to produce the sensors disclosed herein avoid these problems associated with electrodeposition.

Sensors generated by processes such as spin coating processes also avoid other problems associated with electrodeposition, such as those pertaining to the material stresses placed on the sensor during the electrodeposition process. In particular, the process of electrodeposition is observed to produce mechanical stresses on the sensor, for example mechanical stresses that result from tensile and/or compression forces. In certain contexts, such mechanical stresses may result in sensors having coatings with some tendency to crack or delaminate. This is not observed in coatings disposed on sensor via spin coating or other low-stress processes. Consequently, yet another embodiment of the invention is a method of avoiding the electrodeposition influenced cracking and or delamination of a coating on a sensor comprising applying the coating via a spin coating process.

Subsequent to treatment of the sensor elements, one or more additional functional coating or cover layers can then be applied by any one of a wide variety of methods known in the art, such as spraying, dipping, etc. Preferred embodiments of the present invention include an analyte modulating layer deposited over the enzyme-containing layer. In addition to its use in modulating the amount of analyte(s) that contacts the active sensor surface, by utilizing an analyte limiting membrane layer, the problem of sensor fouling by extraneous materials is also obviated. As is known in the art, the thickness of the analyte modulating membrane layer can influence the amount of analyte that reaches the active enzyme. Consequently, its application is preferably carried out under defined processing conditions, and its dimensional thickness is closely controlled. As in the microfabrication of the underlying layers can be a factor which affects close dimensional control over the analyte modulating membrane layer is the composition of the analyte limiting membrane layer material itself. In this regard, it has been discovered that several types of copolymers, for example, a copolymer of a siloxane and a nonsiloxane moiety, are particularly useful. These materials can be microdispensed or spin-coated to a controlled thickness. Their final architecture may also be designed by patterning and photolithographic techniques in conformity with the other discrete structures described herein. Examples of these nonsiloxane-siloxane copolymers include, but are not limited to, dimethylsiloxane-alkene oxide, tetramethyldisiloxane-divinylbenzene, tetramethyldisiloxane-ethylene, dimethylsiloxane-silphenylene, dimethylsiloxane-silphenylene oxide, dimediylsiloxane-a-methylstyrene, dimethylsiloxane-bisphenol A carbonate copolymers, or suitable combinations thereof. The percent by weight of the nonsiloxane component of the copolymer can be preselected to any useful value but typically this proportion lies in the range of about 40-80 wt %. Among the copolymers listed above, the dimethylsiloxane-bisphenol A carbonate copolymer which comprises 50-55 wt % of the nonsiloxane component is preferred. These materials may be purchased from Petrarch Systems, Bristol, Pa. (USA) and are described in this company's products catalog. Other materials which may serve as analyte limiting membrane layers include, but are not limited to, polyurethanes, cellulose acetate, cellulose nitrate, silicone rubber, or combinations of these materials including the siloxane nonsiloxane copolymer, where compatible.

In preferred embodiments of the invention, the sensor is made by methods which apply an analyte modulating layer that comprises a hydrophilic membrane coating which can regulate the amount of analyte that can contact the enzyme of the sensor layer. For example, the cover layer that is added to the glucose sensors of the invention can comprise a glucose limiting membrane, which regulates the amount of glucose that contacts glucose oxidase enzyme layer on an electrode. Such glucose limiting membranes can be made from a wide variety of materials known to be suitable for such purposes, e.g., silicones such as polydimethyl siloxane and the like, polyurethanes, cellulose acetates, Nafion, polyester sulfonic acids (e.g. Kodak AQ), hydrogels or any other membrane known to those skilled in the art that is suitable for such purposes. In certain embodiments of the invention pertaining to sensors having hydrogen peroxide recycling capabilities, the membrane layer that is disposed on the glucose oxidase enzyme layer functions to inhibit the release of hydrogen peroxide into the environment in which the sensor is placed and to facilitate the contact between the hydrogen peroxide molecules and the electrode sensing elements.

In some embodiments of the methods of invention, an adhesion promoter layer is disposed between a cover layer (e.g. an analyte modulating membrane layer) and a sensor chemistry layer in order to facilitate their contact and is selected for its ability to increase the stability of the sensor apparatus. As noted herein, compositions of the preferred adhesion promoter layer are selected to provide a number of desirable characteristics in addition to an ability to provide sensor stability. For example, preferred compositions for use in the adhesion promoter layer are selected to play a role in interference rejection as well as to control mass transfer of the desired analyte. The adhesion promoter layer can be made from any one of a wide variety of materials known in the art to facilitate the bonding between such layers and can be applied by any one of a wide variety of methods known in the art. Preferably, the adhesion promoter layer comprises a silane compound such as γ-aminopropyltrimethoxysilane. In certain embodiments of the invention, the adhesion promoting layer and/or the analyte modulating layer comprises an agent selected for its ability to crosslink a siloxane moiety present in a proximal. In other embodiments of the invention, the adhesion promoting layer and/or the analyte modulating layer comprises an agent selected for its ability to crosslink an amine or carboxyl moiety of a protein present in a proximal layer. In an optional embodiment, the AP layer further comprises Polydimethyl Siloxane (PDMS), a polymer typically present in analyte modulating layers such as a glucose limiting membrane. In illustrative embodiments the formulation comprises 0.5-20% PDMS, preferably 5-15% PDMS, and most preferably 10% PDMS. The addition of PDMS to the AP layer can be advantageous in contexts where it diminishes the possibility of holes or gaps occurring in the AP layer as the sensor is manufactured.

As noted above, a coupling reagent commonly used for promoting adhesion between sensor layers is γ-aminopropyltrimethoxysilane. The silane compound is usually mixed with a suitable solvent to form a liquid mixture. The liquid mixture can then be applied or established on the wafer or planar sensing device by any number of ways including, but not limited to, spin-coating, dip-coating, spray-coating, and microdispensing. The microdispensing process can be carried out as an automated process in which microspots of material are dispensed at multiple preselected areas of the device. In addition, photolithographic techniques such as "lift-off" or using a photoresist cap may be used to localize and define the geometry of the resulting permselective film (i.e. a film having a selective permeability). Solvents suitable for use in forming the silane mixtures include aqueous as well as water-miscible organic solvents, and mixtures thereof. Alcoholic water-miscible organic solvents and aqueous mixtures thereof are particularly useful. These solvent mixtures may further comprise nonionic surfactants, such as polyethylene glycols (PEG) having a for example a molecular weight in the range of about 200 to about 6,000. The addition of these surfactants to the liquid mixtures, at a concentration of about 0.005 to about 0.2 g/dL of the mixture, aids in planarizing the resulting thin films. Also, plasma treatment of the wafer surface prior to the application of the silane reagent can provide a modified surface which promotes a more planar established layer. Water-immiscible organic solvents may also be used in preparing solutions of the silane compound. Examples of these organic solvents include, but are not limited to, diphenylether, benzene, toluene, methylene chloride, dichloroethane, trichloroethane, tetrachloroethane, chlorobenzene, dichlorobenzene, or mixtures thereof. When protic solvents or mixtures thereof are used, the water eventually causes hydrolysis of the alkoxy groups to yield organosilicon hydroxides (especially when n=1) which condense to form poly(organosiloxanes). These hydrolyzed silane reagents are also able to condense with polar groups, such as hydroxyls, which may be present on the substrate surface. When aprotic solvents are used, atmospheric moisture may be sufficient to hydrolyze the alkoxy groups present initially on the silane reagent. The R' group of the silane compound (where n=1 or 2) is chosen to be functionally compatible with the additional layers which are subsequently applied. The R' group usually contains a terminal amine group useful for the covalent attachment of an enzyme to the substrate surface (a compound, such as glutaraldehyde, for example, may be used as a linking agent as described by Murakami, T. et al., Analytical Letters 1986, 19, 1973-86).

Like certain other coating layers of the sensor, the adhesion promoter layer can be subjected to one or more suitable radiation and/or chemical and/or heat curing steps as are known in the art. In alternative embodiments, the enzyme layer can be sufficiently crosslinked or otherwise prepared to allow the membrane cover layer to be disposed in direct contact with the sensor chemistry layer in the absence of an adhesion promoter layer.

A preferred embodiment of the invention is a method of making a sensor by providing a base layer, forming a sensor layer on the base layer, spin coating an enzyme layer on the sensor layer and then forming an analyte contacting layer (e.g. an analyte modulating layer such as a glucose limiting membrane) on the sensor, wherein the analyte contacting layer regulates the amount of analyte that can contact the enzyme layer. In preferred methods, the enzyme layer is vapor crosslinked on the sensor layer. In a typical embodiment of the invention, the sensor layer is formed to include at least one working electrode and at least one counter electrode. In highly preferred embodiments, the enzyme layer is formed on at least a portion of the working electrode and at least a portion of the counter electrode. Typically, the enzyme layer that is formed on the sensor layer is less than 2, 1, 0.5, 0.25 or 0.1 microns in thickness. Preferably, the enzyme layer comprises one or more enzymes such as glucose oxidase, glucose dehydrogenase, lactate oxidase, hexokinase or lactose dehydrogenase and/or like enzymes. In a specific method, the enzyme layer comprises glucose oxidase that is stabilized by coating it on the sensor layer in combination with a carrier protein in a fixed radio. Typically the carrier protein is albumin. Preferably such methods include the step of forming an adhesion promoter layer disposed between the glucose oxidase layer and the analyte contacting layer. Optionally, the adhesion promoter layer is subjected to a curing process prior to the formation of the analyte contacting layer.

A related embodiment of the invention is a method of making a glucose sensor by providing a base layer, forming a sensor layer on the base layer that includes at least one working electrode and at least one counter electrode, forming a glucose oxidase layer on the sensor layer by a spin coating process (a layer which is preferably stabilized by combining the glucose oxidase with albumin in a fixed ratio), wherein the glucose oxidase layer coats at least a portion of the working electrode and at least a portion of the counter electrode, and then forming a glucose limiting layer on the glucose sensor so as to regulate the amount of glucose that can contact the glucose oxidase layer. In such processes, the glucose oxidase layer that is formed on the sensor layer is preferably less than 2, 1, 0.5, 0.25 or 0.1 microns in thickness. Typically, the glucose oxidase coating is vapor crosslinked on the sensor layer. Optionally, the glucose oxidase coating covers the entire sensor layer. In highly preferred embodiments of the invention, an adhesion promoter layer disposed between the glucose oxidase layer and the analyte contacting layer. In certain embodiments of the invention, the analyte sensor further comprises one or more cover layers which are typically electrically insulating protective layers (see, e.g. element 106 in Figure 2). Typically, such cover layers are disposed on at least a portion of the analyte modulating layer.

The finished sensors produced by such processes are typically quickly and easily removed from a supporting substrate (if one is used), for example, by cutting along a line surrounding each sensor on the substrate. The cutting step can use methods typically used in this art such as those that include a UV laser cutting device that is used to cut through the base and cover layers and the functional coating layers along a line surrounding or circumscribing each sensor, typically in at least slight outward spaced relation from the conductive elements so that the sufficient interconnected base and cover layer material remains to seal the side edges of the finished sensor. In addition, dicing techniques typically used to cut ceramic substrates can be used with the appropriate sensor embodiments. Since the base layer is typically not physically attached or only minimally adhered directly to the underlying supporting substrate, the sensors can be lifted quickly and easily from the supporting substrate, without significant further processing steps or potential damage due to stresses incurred by physically pulling or peeling attached sensors from the supporting substrate. The supporting substrate can thereafter be cleaned and reused, or otherwise discarded. Alternatively, the functional coating layer(s) can be applied after the sensor including base layer, sensor elements and cover layer is moved from the supporting substrate by cutting.

### III. METHODS FOR USING ANALYTE SENSOR APPARATUS OF THE INVENTION

A method of sensing an analyte within the body of a mammal is described, the method comprising implanting an analyte sensor embodiment disclosed herein in to the mammal and then sensing an alteration in current at the working electrode and correlating the alteration in current with the presence of the analyte, so that the analyte is sensed. Typically the analyte sensor is polarized anodically such that the working electrode where the alteration in current is sensed is an anode. In one such method, the analyte sensor apparatus senses glucose in the mammal. In an alternative method, the analyte sensor apparatus senses lactate, potassium, calcium, oxygen, pH, and/or any physiologically relevant analyte in the mammal.

Certain analyte sensors having the structure discussed above have a number of highly desirable characteristics which allow for a variety of methods for sensing analytes in a mammal. For example in such methods, the analyte sensor apparatus implanted in the mammal functions to sense an analyte within the body of a mammal for more than 1, 2, 3, 4, 5, or 6 months. Preferably, the analyte sensor apparatus so implanted in the mammal senses an alteration in current in response to an analyte within 15, 10, 5 or 2 minutes of the analyte contacting the sensor. In such methods, the sensors can be implanted into a variety of locations within the body of the mammal, for example in both vascular and non-vascular spaces.

### IV. KITS AND SENSOR SETS OF THE INVENTION

In another embodiment of the invention, a kit and/or sensor set, useful for the sensing an analyte as is described above, is provided. The kit and/or sensor set typically comprises a container, a label and an analyte sensor as described above. Suitable containers include, for example, an easy to open package made from a material such as a metal foil, bottles, vials, syringes, and test tubes. The containers may be formed from a variety of materials such as metals (e.g. foils) paper products, glass or plastic. The label on, or associated with, the container indicates that the sensor is used for assaying the analyte of choice. In preferred embodiments, the container holds a glucose sensor coated with a layer of glucose oxidase that is less than 2 microns in thickness. The kit and/or sensor set may further include other materials desirable from a commercial and user standpoint, including elements or devices designed to facilitate the introduction of the sensor into the analyte environment, other buffers, diluents, filters, needles, syringes, and package inserts with instructions for use.

## Claims

1. An analyte sensor apparatus (100) for implantation within a mammal, the analyte sensor apparatus comprising:
a base layer (102);
a conductive layer (104) disposed upon the base layer wherein the conductive layer (104) includes a working electrode;
an analyte sensing layer (110) disposed on the conductive layer (104), wherein the analyte sensing layer (110) detectably alters the electrical current at the working electrode in the conductive layer (104) in the presence of an analyte;
means for providing anodic polarization such that the alteration in current is detected at the anode working electrode in the conductive layer of the analyte sensor apparatus, the alteration in current being correlated with the concentration of the analyte;
**characterized by**
a protein layer (116) disposed on said analyte sensing layer (110);
an adhesion promoting layer (114) disposed on said protein layer (116), and a analyte modulating layer (112) disposed on said adhesion promoting layer (114).

2. The analyte sensor apparatus (100) of claim 1, wherein the adhesion promoting layer (114) comprises a silane composition.

3. The analyte sensor apparatus (100) of claim 1, wherein the adhesion promoting layer (114) comprises polydimethyl siloxane.

4. The analyte sensor apparatus (100) of claim 1, wherein a protein within the protein layer (116) is an albumin selected from the group consisting of bovine serum albumin and human serum albumin.

5. The analyte sensor apparatus (100) of claim 1, further comprising a cover layer (106) disposed on at least a portion of the analyte modulating layer (112), wherein the cover layer (106) further includes an aperture (108) that exposes at least a portion of the analyte modulating (112) layer to a solution comprising the analyte to be sensed.

6. The analyte sensor apparatus (100) of claim 1, wherein the analyte sensing layer (110) comprises an enzyme selected from the group consisting of glucose oxidase, glucose dehydrogenase, lactate, oxidase, hexokinase and lactose dehydrogenase.

7. The analyte sensor apparatus (100) of claim 6, wherein the enzyme layer further comprises a carrier protein in a substantially fixed ratio with the enzyme.

8. The analyte sensor apparatus (100) of claim 7, wherein the enzyme and the carrier protein are distributed in a substantially uniform manner throughout the enzyme layer.

9. The analyte sensor apparatus (100) of claim 6, wherein the enzyme layer is a thickness selected from the group consisting of less than 1, 0.5, 0.25 and 0.1 microns.

10. The analyte sensor apparatus (100) of claim 10, wherein the enzyme is glucose oxidase and the analyte sensor apparatus (100) is capable of sensing glucose levels in the mammal.

11. The analyte sensor apparatus of claim 10, wherein the current at the working anode electrode in the conductive layer (104) is altered by hydrogen peroxide that is generated from the enzymatic reaction between glucose and glucose oxidase.

12. The analyte sensor apparatus (100) of claim 9, wherein the enzyme is lactate oxidase and the analyte sensor apparatus (100) is capable of sensing lactate levels in the mammal.

13. The analyte sensor apparatus (100) of claim 12, wherein the working anode electrode in the conductive layer(104) is altered by hydrogen peroxide that is generated from the enzymatic reaction between lactate and lactate oxidase.

14. The analyte sensor apparatus (100) of claim 1, wherein the conductive layer (104) further comprises a counter electrode or a reference electrode.

15. The analyte sensor apparatus (100) of claim 1, wherein the analyte sensor apparatus (100) is suitable for implantation in the mammal for a time period of greater than 30 days.

16. The analyte sensor apparatus (100) of claim 1, wherein the analyte sensor apparatus (100) is suitable for implantation in the mammal in a non-vascular space.

17. The analyte sensor apparatus (100) of claim 1, wherein the alteration in current in response to exposure to the analyte present in the body of the mammal can be detected via an amperometer within 15, 10, 5 or 2 minutes of the analyte contacting the sensor.

18. The analyte sensor apparatus (100) of claim 1, wherein the analyte sensor apparatus (100) is of a planar geometry.

19. A method of making a sensor apparatus for implantation within a mammal comprising the steps of:
providing a base layer;
forming a conductive layer on the base layer, wherein the conductive layer includes a working electrode;
forming an analyte sensing layer on the conductive layer, wherein the analyte sensing layer includes a composition that can alter the electrical current at the working electrode in the conductive layer in the presence of an analyte;
forming a protein layer on the analyte sensing layer;
forming an adhesion promoting layer on the analyte sensing layer or the optional protein layer;
forming an analyte modulating layer disposed on the adhesion promoting layer, wherein the analyte modulating layer includes a composition that modulates the diffusion of the analyte therethrough; and
forming a cover layer disposed on at least a portion of the analyte modulating layer, wherein the cover layer further includes an aperture over at least a portion of the analyte modulating layer.

20. The method of claim 19 wherein the adhesion promoting layer includes a silane composition.

21. The method of claim 19, wherein the analyte sensing layer is formed by a spin coating process.

22. The method of claim 19, wherein the analyte sensing layer is a thickness selected from the group consisting of less than 1, 0.5, 0.25 and 0.1 microns.

23. The method of claim 19, wherein the method includes the step of forming a protein layer on the analyte sensing layer, wherein a protein within the protein layer is an albumin selected from the group consisting of bovine serum albumin and human serum albumin.

24. The method of claim 19, wherein the analyte sensing layer comprises a enzyme composition selected from the group cnsisting of glucose oxidase, glucose dehydrogenase, lactate oxidase, hexokinase and lactose dehydrogenase.

25. The method of claim 24, wherein the analyte sensing layer further comprises a carrier protein composition in a substantially fixed ratio with the enzyme and the enzyme and the carrier protein are distributed in a substantially uniform manner throughout the analyte sensing layer.

26. The method of claim 19, wherein the conductive layer further comprises a counter electrode or a reference electrode.

27. The method of claim 25, wherein the enzyme is glucose oxidase and the sensor is designed to sense alteration in electrical current at the working electrode that occur in the presence of changing hydrogen peroxide concentrations that result from the reaction between glucose oxidase and glucose.

28. The method of claim 19, wherein the analyte sensor apparatus is formed in a planar geometric configuration.

29. A kit comprising a container and, within the container, an analyte sensor apparatus according to claim 1 and instructions for using the analyte sensor apparatus.

## Patentansprüche

1. Analytsensorvorrichtung (100) zur Implantation in Säugetiere, die Analytsensorvorrichtung umfasst:
eine Basisschicht (102);
eine leitfähige Schicht (104), die auf der Basisschicht angeordnet ist, wobei die leitfähige Schicht (104) eine Arbeitselektrode einschließt;
eine Analyt detektierende Schicht (110), die auf der leitfähigen Schicht (104) angeordnet ist, wobei die Analyt detektierende Schicht (110) nachweisbar den elektrischen Strom an der Arbeitselektrode in der leitfähigen Schicht (104) in Gegenwart des Analyten ändert;
Mittel zur Herstellung einer anodischen Polarisation, so dass die Änderung des Stromes an der anodischen Arbeitselektrode in der leitfähigen Schicht der Analytsensorvorrichtung nachgewiesen wird, die Änderung des Stromes ist korreliert mit der Konzentration des Analyten; **dadurch gekennzeichnet dass**,
eine Proteinschicht (116) auf der Analyt detektierende Schicht (110) angeordnet ist;
eine adhäsionsfördernde Schicht (114) ist auf der Proteinschicht (116) angeordnet, und eine Analytmodulationsschicht (112) ist auf der adhäsionsfördernden Schicht (114) angeordnet.

2. Die Analytsensorvorrichtung (100) nach Anspruch 1, bei der die adhäsionsfördernde Schicht (114) eine Silanzusammensetzung umfasst.

3. Die Analytsensorvorrichtung (100) nach Anspruch 1, bei der die adhäsionsfördernde Schicht (114) Polydimethylsiloxan umfasst.

4. Die Analytsensorvorrichtung (100) nach Anspruch 1, bei der ein Protein innerhalb der Proteinschicht (116) ein Albumin ist, ausgewählt aus der
Gruppe bestehend aus Rinderserumalbumin und humanem Serumalbumin.

5. Die Analytsensorvorrichtung (100) nach Anspruch 1, die des weiteren eine Deckschicht (106) umfasst, die auf wenigstens einem Teil der Analytmodulationsschicht (112) angeordnet ist, wobei die Deckschicht (106) weiterhin eine Öffnung (108) einschließt, die wenigstens einen Teil der Analytmodulationsschicht (112) einer Lösung exponiert, die den zu nachzuweisenden Analyten umfasst.

6. Die Analytsensorvorrichtung (100) nach Anspruch 1, bei der die Analyt detektierende Schicht (110) ein Enzym, ausgewählt aus der Gruppe bestehend aus Glucoseoxidase, Glucosedehydrogenase, Lactatoxidase, Hexokinase und Lactosedehydrogenase, umfasst.

7. Die Analytsensorvorrichtung (100) nach Anspruch 6, bei der die Enzymschicht des weiteren ein Trägerprotein in einem im wesentlichen fixierten Verhältnis mit dem Enzym umfasst.

8. Die Analytsensorvorrichtung (100) nach Anspruch 7, bei der das Enzym und das Trägerprotein in einer im wesentlichen einheitlichen Weise innerhalb der Enzymschicht verteilt sind.

9. Die Analytsensorvorrichtung (100) nach Anspruch 6, bei der die Enzymschicht eine Dicke ausgewählt aus der Gruppe von weniger als 1, 0,5, 0,25 und 0,1 µm aufweist.

10. Die Analytsensorvorrichtung (100) nach Anspruch 9, bei der das Enzym Glucoseoxidase ist und die Analytsensorvorrichtung (100) in der Lage ist, den Glucosegehalt in Säugetieren nachzuweisen.

11. Die Analytsensorvorrichtung (100) nach Anspruch 10, bei der der Strom an der anodischen Arbeitselektrode in der leitfähigen Schicht (104) durch Wasserstoffperoxid geändert wird, das durch die enzymatische Reaktion zwischen Glucose und Glucoseoxidase erzeugt wird.

12. Die Analytsensorvorrichtung (100) nach Anspruch 9, bei der das Enzym Lactatoxidase ist und die Analytsensorvorrichtung (100) in der Lage ist, den Lactatgehalt in Säugetieren nachzuweisen.

13. Die Analytsensorvorrichtung (100) nach Anspruch 12, bei der die anodische Arbeitselektrode in der leitfähigen Schicht (104) durch Wasserstoffperoxid geändert wird, das durch die enzymatische Reaktion zwischen Lactat und Lactatoxidase erzeugt wird.

14. Die Analytsensorvorrichtung (100) nach Anspruch 1, bei der die leitfähige Schicht (104) des weiteren eine Gegenelektrode oder eine Referenzelektrode umfasst.

15. Die Analytsensorvorrichtung (100) nach Anspruch 1, bei der die Analytsensorvorrichtung (100) zur Implantation in Säugetiere über einen Zeitraum von mehr als 30 Tagen geeignet ist.

16. Die Analytsensorvorrichtung (100) nach Anspruch 1, bei der die Analytsensorvorrichtung (100) zur Implantation in Säugetiere in einem nicht-vaskulären Bereich geeignet ist.

17. Die Analytsensorvorrichtung (100) nach Anspruch 1, bei der die Änderung des Stroms als Antwort auf die Exposition des Analyten, der im Körper von Säugetieren vorhanden ist, mittels eines Amperometers innerhalb von 15, 10, 5 oder 2 Minuten, nachdem der Analyt mit dem Sensor in Berührung gekommen ist, nachgewiesen werden kann.

18. Die Analytsensorvorrichtung (100) nach Anspruch 1, bei der die Analytsensorvorrichtung (100) eine planare Geometrie aufweist.

19. Verfahren zur Herstellung einer Analytsensorvorrichtung zur Implantation in Säugetiere umfassend die Schritte:
Bereitstellen einer Basisschicht;
Bilden einer leitfähigen Schicht auf der Basisschicht, wobei die leitfähige Schicht eine Arbeitselektrode einschließt;
Bilden einer Analyt detektierende Schicht auf der leitfähigen Schicht, wobei die Analyt detektierende Schicht eine Zusammensetzung einschließt, die eine elektrischen Strom an der Arbeitselektrode in der leitfähigen Schicht in Gegenwart eines Analyten ändern kann;
Bilden einer Proteinschicht auf der Analyt detektierende Schicht ;
Bilden einer adhäsionsfördernden Schicht auf der Analyt detektierende Schicht oder optionalen Proteinschicht;
Bilden einer Analytmodulationsschicht, die auf der adhäsionsfördernden Schicht angeordnet ist, wobei die Analytmodulationsschicht eine Zusammensetzung einschließt, die die Diffusion des Analyten durch diese moduliert; und
Bilden einer Deckschicht, die auf wenigstens einem Teil der Analytmodulationsschicht angeordnet ist, wobei die Deckschicht des weiteren eine Öffnung über einen Teil der Analytmodulationsschicht einschließt.

20. Verfahren nach Anspruch 19, bei dem die adhäsionsfördernde Schicht eine Silanzusammensetzung umfasst.

21. Verfahren nach Anspruch 19, bei dem die Analyt detektierende Schicht durch ein Spin-Beschichtungsverfahren gebildet wird.

22. Verfahren nach Anspruch 19, bei dem die Analyt detektierende Schicht eine Dicke hat ausgewählt aus der Gruppe bestehend aus weniger als 1, 0,5, 0,25 und 0,1 µm.

23. Verfahren nach Anspruch 19, wobei das Verfahren den Schritt der Bildung einer Proteinschicht auf der Analyt detektierende Schicht (110) einschließt, wobei ein Protein in der Proteinschicht ein Albumin ist, ausgewählt aus der Gruppe bestehend aus Rinderserumalbumin und humanem Serumalbumin.

24. Verfahren nach Anspruch 19, bei dem die Analyt detektierende Schicht eine Enzymzusammensetzung umfasst, die ausgewählt wird aus der Gruppe bestehend aus Glucoseoxidase, Glucosedehydrogenase, Lactatoxidase, Hexokinase und Lactosedehydrogenase.

25. Verfahren nach Anspruch 24, bei dem Analyt detektierende Schicht des weiteren ein Trägerprotein in einem im wesentlichen fixierten Verhältnis mit dem Enzym umfasst und das Enzym und das Trägerprotein in einer im wesentlichen einheitlichen Weise innerhalb der Analyt detektierende Schicht verteilt sind.

26. Verfahren nach Anspruch 19, bei dem die leitfähige Schicht eine Gegenelektrode oder eine Referenzelektrode umfasst.

27. Verfahren nach Anspruch 25, bei dem das Enzym Glucoseoxidase ist und der Sensor so ausgebildet ist, um die Änderungen des elektrischen Stroms an der Arbeitselektrode nachzuweisen, die in Gegenwart von wechselnden Wasserstoffperoxidkonzentrationen, die aus der Reaktion zwischen Glucoseoxidase und Glucose resultieren, auftreten.

28. Verfahren nach Anspruch 19, bei dem die Analytsensorvorrichtung in einer planaren geometrischen Konfiguration gebildet ist.

29. Ein Kit umfassend einen Behälter und innerhalb des Behälters eine Analytsensorvorrichtung nach Anspruch 1 und Anweisungen zur Verwendung der Analytsensorvorrichtung.

## Revendications

1. Appareil de détection d'analyte (100) pour une implantation à l'intérieur d'un mammifère, l'appareil de détection d'analyte comprenant :
une couche de base (102) ;
une couche conductrice (104) disposée sur la couche de base, dans lequel la couche conductrice (104) comporte une électrode de travail ;
une couche de détection d'analyte (110) disposée sur la couche conductrice (104), dans lequel la couche de détection d'analyte (110) change le courant électrique de manière détectable sur l'électrode de travail dans la couche conductrice (104) en présence d'un analyte ;
des moyens pour produire une polarisation anodique de telle sorte que le changement dans le courant est détecté sur l'électrode d'anode de travail dans la couche conductrice de l'appareil de détection d'analyte, le changement dans le courant étant corrélé avec la concentration de l'analyte ;
**caractérisé par**
une couche de protéine (116) disposée sur ladite couche de détection d'analyte (110) ;
une couche favorisant l'adhérence (114) disposée sur ladite couche de protéine (116), et une couche de modulation d'analyte (112) disposée sur ladite couche favorisant l'adhérence (114).

2. Appareil de détection d'analyte (100) selon la revendication 1, dans lequel la couche favorisant l'adhérence (114) comprend une composition de silane.

3. Appareil de détection d'analyte (100) selon la revendication 1, dans lequel la couche favorisant l'adhérence (114) comprend du polydiméthylsiloxane.

4. Appareil de détection d'analyte (100) selon la revendication 1, dans lequel une protéine à l'intérieur de la couche de protéine (116) est une albumine choisie parmi le groupe constitué d'une albumine de sérum bovin et d'une albumine de sérum humain.

5. Appareil de détection d'analyte (100) selon la revendication 1, comprenant en outre une couche de recouvrement (106) disposée sur au moins une partie de la couche de modulation d'analyte (112), dans lequel la couche de recouvrement (106) comporte en outre une ouverture (108) qui expose au moins une partie de la couche de modulation d'analyte (112) à une solution comprenant l'analyte à détecter.

6. Appareil de détection d'analyte (100) selon la revendication 1, dans lequel la couche de détection d'analyte (110) comprend une enzyme choisie parmi le groupe constitué de la glucose oxydase, de la glucose déshydrogénase, de la lactate oxydase, de l'hexokinase et de la lactose déshydrogénase.

7. Appareil de détection d'analyte (100) selon la revendication 6, dans lequel la couche d'enzyme comprend en outre une protéine porteuse dans un rapport sensiblement fixe avec l'enzyme.

8. Appareil de détection d'analyte (100) selon la revendication 7, dans lequel l'enzyme et la protéine porteuse sont réparties de manière sensiblement uniforme à travers toute la couche d'enzyme.

9. Appareil de détection d'analyte (100) selon la revendication 6, dans lequel la couche d'enzyme est d'une épaisseur choisie parmi le groupe constitué de moins de 1, 0,5, 0,25 et 0,1 micron.

10. Appareil de détection d'analyte (100) selon la revendication 10, dans lequel l'enzyme est la glucose oxydase et l'appareil de détection d'analyte (100) est capable de détecter des niveaux de glucose dans le mammifère.

11. Appareil de détection d'analyte selon la revendication 10, dans lequel le courant sur l'électrode d'anode de travail dans la couche conductrice (104) est changé par du peroxyde d'hydrogène qui est généré à partir de la réaction enzymatique entre le glucose et la glucose oxydase.

12. Appareil de détection d'analyte (100) selon la revendication 9, dans lequel l'enzyme est la lactate oxydase et l'appareil de détection d'analyte (100) est capable de détecter des niveaux de lactate dans le mammifère.

13. Appareil de détection d'analyte (100) selon la revendication 12, dans lequel l'électrode d'anode de travail dans la couche conductrice (104) est changée par du peroxyde d'hydrogène qui est généré à partir de la réaction enzymatique entre le lactate et la lactate oxydase.

14. Appareil de détection d'analyte (100) selon la revendication 1, dans lequel la couche conductrice (104) comprend en outre une contre-électrode ou une électrode de référence.

15. Appareil de détection d'analyte (100) selon la revendication 1, dans lequel l'appareil de détection d'analyte (100) est adapté pour une implantation dans le mammifère pendant une période de temps supérieure à 30 jours.

16. Appareil de détection d'analyte (100) selon la revendication 1, l'appareil de détection d'analyte étant adapté pour une implantation dans le mammifère dans un espace non vasculaire.

17. Appareil de détection d'analyte (100) selon la revendication 1, dans lequel le changement de courant en réponse à une exposition à l'analyte présent dans le corps du mammifère peut être détecté via un ampèremètre pendant les 15, 10, 5 ou 2 minutes où l'analyte est en contact avec le détecteur.

18. Appareil de détection d'analyte (100) selon la revendication 1, dans lequel l'appareil de détection d'analyte (100) est d'une géométrie plane.

19. Procédé de fabrication d'un appareil de détection pour une implantation à l'intérieur d'un mammifère comprenant les étapes de :
fourniture d'une couche de base ;
formation d'une couche conductrice sur la couche de base, la couche conductrice comportant une électrode de travail ;
formation d'une couche de détection d'analyte sur la couche conductrice, la couche de détection d'analyte comportant une composition qui peut changer le courant électrique sur l'électrode de travail dans la couche conductrice en présence d'un analyte ;
formation d'une couche de protéine sur la couche de détection d'analyte ;
formation d'une couche favorisant l'adhérence sur la couche de détection d'analyte ou la couche de protéine facultative ;
formation d'une couche de modulation d'analyte disposée sur la couche favorisant l'adhérence, la couche de modulation d'analyte comportant une composition qui module la diffusion de l'analyte à travers celle-ci ; et
formation d'une couche de recouvrement disposée sur au moins une partie de la couche de modulation d'analyte, la couche de recouvrement comportant en outre une ouverture sur au moins une partie de la couche de modulation d'analyte.

20. Procédé selon la revendication 19, dans lequel la couche favorisant l'adhérence comporte une composition de silane.

21. Procédé selon la revendication 19, dans lequel la couche de détection d'analyte est formée par un procédé d'enduction par centrifugation.

22. Procédé selon la revendication 19, dans lequel la couche de détection d'analyte est d'une épaisseur choisie parmi le groupe constitué de moins de 1, 0,5, 0,25 et 0,1 micron.

23. Procédé selon la revendication 19, le procédé comportant les étapes de formation d'une couche de protéine sur la couche de détection d'analyte, dans lequel une protéine à l'intérieur de la couche de protéine est une albumine choisie parmi le groupe constitué d'une albumine de sérum bovin et d'une albumine de sérum humain.

24. Procédé selon la revendication 19, dans lequel la couche de détection d'analyte comprend une composition d'enzyme choisie parmi le groupe constitué de la glucose oxydase, de la glucose déshydrogénase, de la lactate oxydase, de l'hexokinase et de la lactose déshydrogénase.

25. Procédé selon la revendication 24, dans lequel la couche de détection d'analyte comprend en outre une composition de protéine porteuse dans un rapport sensiblement fixe avec l'enzyme, et l'enzyme et la protéine porteuse sont réparties de manière sensiblement uniforme à travers toute la couche de détection d'analyte.

26. Procédé selon la revendication 19, dans lequel la couche conductrice comprend en outre une contre-électrode ou une électrode de référence.

27. Procédé selon la revendication 25, dans lequel l'enzyme est la glucose oxydase et le détecteur est conçu pour détecter des changements dans le courant électrique sur l'électrode de travail qui se produisent en présence d'une variation de concentrations de peroxyde d'hydrogène qui résultent de la réaction entre la glucose oxydase et le glucose.

28. Procédé selon la revendication 19, dans lequel l'appareil de détection d'analyte est formé dans une configuration géométrique plane.

29. Kit comprenant un récipient et, à l'intérieur du récipient, un appareil de détection d'analyte selon la revendication 1 et des instructions pour utiliser l'appareil de détection d'analyte.
